# EUROPEAN PATENT APPLICATION

(11) **EP 4 678 185 A1**
(43) Date of publication of application: **14.01.2026**
(21) Application number: 24766381.8
(22) Date of filing: 01.03.2024
(51) Int. Cl.: A61K 39/12, C12N 15/37, A61P 31/20

(54) **HUMAN PAPILLOMAVIRUS VACCINE AND USE THEREOF**

(30) Priority: 07.03.2023 CN 202310212610
(71) Applicant: Beijing Health Guard Biotechnology, Inc., Beijing 100176 (CN)
(72) Inventor: YIN, Fei, Beijing 100176 (CN); WU, Shuming, Beijing 100176 (CN); LIU, Yuying, Beijing 100176 (CN); ZHANG, Haijiang, Beijing 100176 (CN); JIANG, Xulin, Beijing 100176 (CN); SHEN, Ercui, Beijing 100176 (CN); ZHANG, Chunyan, Beijing 100176 (CN); WANG, Yan, Beijing 100176 (CN); LI, Ling, Beijing 100176 (CN); CHEN, Xiao, Beijing 100176 (CN); GAO, Wenshuang, Beijing 100176 (CN); CHEN, Dan, Beijing 100176 (CN); YANG, Xiufen, Beijing 100176 (CN); ZHANG, Ruixia, Beijing 100176 (CN); LIU, Yongjiang, Beijing 100176 (CN); YU, Hongyang, Beijing 100176 (CN)
(74) Representative: Karakatsanis, Georgios
(86) International application number: PCT/CN2024/079758
(87) International publication number: WO 2024/183665

(57) **Abstract**

Provided is a human papillomavirus (HPV) vaccine, including combinations of L1 protein antigens of HPV6, 11, 16, 18, 31, 33, 35, 39, 45, 51, 52, 56, 58, 59, and 68. A recombinant 15-valent human papillomavirus vaccine contains the above 12 types clearly associated with human cancers, as well as two most common low-risk types: HPV6 and 11, and a suspected carcinogenic type: HPV68. In China, it is expected that the preventive effect of this vaccine on cervical cancer in women will increase to 97.2% or more, while globally, the preventive effect thereof on cervical cancer in women will be expected to increase to 94.1% or more, with significant application value.

## Description

### TECHNICAL FIELD

The present invention relates to the field of medicine, specifically to a human papillomavirus vaccine, and in particular to a 15-valent human papillomavirus vaccine.

### BACKGROUND

Human papillomavirus (HPV) is a small, non-enveloped deoxyribonucleic acid (DNA) virus that can infect human epidermal and mucosal squamous epithelial cells. The relationship between HPV infection and cervical cancer was first proposed in 1974, and it was ultimately proven that HPV infection is the main cause of cervical cancer. HPV infection is the most common reproductive tract virus infection. Human infection with HPV may not only cause cervical cancer, but also anogenital cancer and genital warts. In addition, the occurrence of oropharyngeal cancer and other head and neck cancers, colon cancer, and rectal cancer is also related to HPV infection. There are more than 200 types of HPV that have been identified so far, which can be divided into low-risk and high-risk types according to their carcinogenicity. Low-risk HPV mainly includes HPV6/11/30/42/43/44/61, and 90% of genital warts are caused by infection with HPV6 and 11. High-risk HPV newly defined by the World Health Organization (WHO) International Agency for Research on Cancer (IARC) includes 12 types (HPV16/18/31/33/35/39/45/51/52/56/58/59) that are clearly associated with human cancers and 2 types (HPV66/68) with limited evidence of carcinogenicity. High-risk HPV is carcinogenic and is the cause of almost all cervical cancers. In addition, it also causes 88% of anal cancers, 78% of vaginal cancers, 15-48% of vulvar cancers (age-related), 51% of penile cancers and 13-60% of oropharyngeal cancers.

HPV vaccination is the most economical and effective means to prevent persistent HPV infection and related diseases. Currently, there are three types of HPV vaccines available overseas: a bivalent HPV vaccine (HPV16/18) (trade name: Cervarix) produced by the GSK Company in Britain, a quadrivalent HPV vaccine (HPV6/11/16/18) (trade name: Gardasil) produced by the Merck Company in the United States, and a recombinant 9-valent HPV vaccine (HPV6/11/16/18/31/33/45/52/58) (trade name: Gardasil9). These three HPV preventive vaccines all use a DNA recombination technology to express and purify HPV L1 structural proteins, and then are self-assembled to form HPV genotype-specific virus-like particles (VLPs). Because VLPs do not contain any viral nucleic acid components and have no protein conversion effect similar to prion proteins, they are not infectious and are only used for prevention.

Up to now, there is no broad-spectrum preventive HPV vaccine on the market in China or abroad that covers all 12 types (HPV16, 18, 31, 33, 35, 39, 45, 51, 52, 56, 58, 59) that are clearly related to human cancers, two types with limited evidence of carcinogenicity (HPV66/68), and HPV6 and 11 that cause genital warts. Therefore, a broader-spectrum HPV vaccine is urgently needed to improve the preventive effect of cervical cancer in women.

### SUMMARY

Based on the demands of the prior art, the present invention has obtained a 15-valent preventive HPV vaccine covering all 12 types (HPV16, 18, 31, 33, 35, 39, 45, 51, 52, 56, 58, and 59) that are clearly associated with human cancers through in-depth research.

First, the present invention provides a human papillomavirus vaccine, including one or more combinations of L1 antigens selected from HPV types 6, 11, 16, 18, 31, 33, 35, 39, 45, 51, 52, 56, 58, 59, and 68, wherein the combination is 15 types, 9 types, 6 types, or 3 types of the L1 antigens;
wherein a weight ratio of amounts of the L1 antigens of HPV types 6, 11, 16, 18, 31, 33, 35, 39, 45, 51, 52, 56, 58, 59, and 68 is (1-3):(1-5):(2-7):(1-5):(0.5-3):(0.5-3):(0.5-3):(0.5-3):(0.5-3):(0.5-2):(0.5-2):(0.5-2):(0.5-2):(0.5-2):(0.5-2); and wherein the weight ratio of the amounts of the L1 antigens of HPV types 6, 11, 16, 18, 31, 33, 35, 39, 45, 51, 52, 56, 58, 59, and 68 is 1.5:2:3:2:1:1:1:1:1:1:1:1:1:1:1, or 1.5:2:3.5:2.5:1:1:1:1:1:1:1:1:1:1:1, or 3:4:6:4:2:2:2:2:2:1:1:1:1:1:1; and
more specifically, the amount of each protein is 10-100 µg, and wherein per 0.5 mL the vaccine contains: HPV type 6 at 30 µg, HPV type 11 at 40 µg, HPV type 16 at 60 µg, HPV type 18 at 40 µg, HPV type 31 at 20 µg, HPV type 33 at 20 µg, HPV type 35 at 20 µg, HPV type 39 at 20 µg, HPV type 45 at 20 µg, HPV type 51 at 20 µg, HPV type 52 at 20 µg, HPV type 56 at 20 µg, HPV type 58 at 20 µg, HPV type 59 at 20 µg, and HPV type 68 at 20 µg.

Further preferably, each of the L1 antigens is truncated relative to the wild-type sequence as follows: wild-type HPV6 L1 is truncated by 2 amino acids at the N-terminus and by 29 amino acids at the C-terminus; wild-type HPV11 L1 is truncated by 3 amino acids at the N-terminus and by 29 amino acids at the C-terminus; wild-type HPV16 L1 is truncated by 4 amino acids at the N-terminus and by 29 amino acids at the C-terminus; wild-type HPV18 L1 is truncated by 4 amino acids at the N-terminus and by 30 amino acids at the C-terminus; wild-type HPV31 L1 is truncated by 4 amino acids at the N-terminus and by 27 amino acids at the C-terminus; wild-type HPV33 L1 is truncated by 4 amino acids at the N-terminus and by 24 amino acids at the C-terminus; wild-type HPV35 L1 is truncated by 4 amino acids at the N-terminus and by 28 amino acids at the C-terminus; wild-type HPV39 L1 is truncated by 9 amino acids at the N-terminus and by 29 amino acids at the C-terminus; wild-type HPV45 L1 is truncated by 4 amino acids at the N-terminus and by 30 amino acids at the C-terminus; wild-type HPV51 L1 is truncated by 4 amino acids at the N-terminus and by 28 amino acids at the C-terminus; wild-type HPV52 L1 is truncated by 4 amino acids at the N-terminus and by 23 amino acids at the C-terminus; wild-type HPV56 L1 is truncated by 4 amino acids at the N-terminus and by 25 amino acids at the C-terminus; wild-type HPV58 L1 is truncated by 4 amino acids at the N-terminus and by 23 amino acids at the C-terminus; wild-type HPV59 L1 is truncated by 4 amino acids at the N-terminus and by 31 amino acids at the C-terminus; and wild-type HPV68 L1 is truncated by 4 amino acids at the N-terminus and by 28 amino acids at the C-terminus.

In a specific embodiment, the L1 antigens of HPV6, 11, 16, 18, 31, 33, 35, 39, 45, 51, 52, 56, 58, 59, and 68 comprise the amino acid sequence of SEQ ID NO: 1 to SEQ ID NO: 15, respectively.

In addition, preferably, an adjuvant used is an aluminum adjuvant, wherein the adjuvant is an aluminum hydroxide adjuvant; and a mass ratio of antigen protein to the aluminum adjuvant is (0.5-1):1, wherein the mass ratio is (0.7-0.85): 1.

Further preferably, a buffer used in the vaccine is an acetic acid-sodium acetate buffer system, and the vaccine comprises an excipient composed of histidine, sodium chloride, and polysorbate 80.

Even further preferably, a pH value of the acetic acid-sodium acetate buffer system is 5-7, wherein the pH value is 5.3-6.5; a concentration of the acetic acid-sodium acetate buffer system is 5-15 mM, wherein the concentration is 10 mM; a concentration of sodium chloride is 100-400 mM, wherein the concentration is 320-335 mM; and a concentration of polysorbate 80 is 0.005%-0.02%, wherein the concentration is 0.01%.

The present invention also provides a method for preparing the vaccine, including the following steps:
purifying L1 proteins of each HPV type, subjecting the purified proteins to self-assembly *in vitro* to form virus-like particles (VLPs), subjecting the VLPs to column chromatography for buffer exchange and sterile filtration to prepare a bulk protein solution, and diluting the bulk protein solution with a buffer solution to a required concentration; and mixing the diluted protein solutions of all types in proportion to form a fifteen-valent protein dilution, which are sterilized and filtered for later use;
taking a prescribed amount of aluminum hydroxide adjuvant, diluting and mixing the aluminum hydroxide adjuvant uniformly (using a magnetic stirrer at 300-340 rpm for not less than 30 minutes), filtering the diluted aluminum hydroxide adjuvant through a capsule filter for later use;
taking a required amount of the sterile-filtered fifteen-valent protein dilution, taking a required amount of the filtered aluminum hydroxide adjuvant dilution, and mixing the protein dilution and adjuvant dilution until completely uniform to obtain a semi-finished product; and
wherein the method further comprising: performing final product filling by: starting a pre-filled syringe filling machine, operating the filling machine at 30-40 rpm, filling 0.55 mL per syringe and stoppering, and storing the stoppered syringes in cold storage for later use.

In a specific embodiment, the method for preparing the purified L1 proteins of each HPV type comprising:
taking a fermentation product of recombinant bacteria expressing the L1 protein of each type, resuspending bacterial cells from the fermentation product, disrupting the resuspended cells using a high-pressure homogenizer, centrifuging the disrupted cell slurry, and collecting a supernatant after centrifugation;
adding ammonium sulfate powder to the supernatant to a saturation of 25-45%, and slowly stirring until the resulting mixture is completely dissolved;
centrifuging continuously, collecting a precipitate, completely resuspending the precipitate, centrifuging the resuspended precipitate, and collecting a supernatant;
filtering the clarified supernatant, performing 2-stage depth filtration, and using filter membranes with pore sizes of 3.0 to 6.0 µm and 0.2 to 0.4 µm; and
performing EQ anion exchange chromatography→SQ anion exchange chromatography→gel filtration chromatography for optimization; wherein the specific operation comprises:
   (1) EQ anion exchange chromatography: after column equilibration, loading a sample collected after deep filtration; performing a wash after loading completion, and collecting an EQ ion exchange flow-through liquid with OD280 greater than 50 mAU;
   (2) SQ anion exchange chromatography: after column equilibration, loading the EQ ion exchange flow-through liquid from the step (1); performing a wash after loading completion; performing an elution after the wash ends, and collecting a target protein absorption peak with OD280 not less than 40 mAU during elution; and
   (3) gel filtration chromatography: after equilibrating a chromatographic column until a baseline is stable, loading a target protein sample collected from SQ anion exchange chromatography elution, and collecting an HPV L1 protein absorption peak with OD280 greater than 50 mAU.

The present invention also provides a method for preserving the vaccine, wherein the vaccine is preserved at 2-8°C.

Finally, the present invention provides a use of the vaccine in the preparation of a drug for preventing or treating diseases caused by human papillomavirus.

Beneficial effects of the present invention: This vaccine has 6 newly added antigens varying in valent types, thus being broader in spectrum. Moreover, after the antigen doses are optimized, it will not cause immunization interference between antigen types. The recombinant 15-valent human papillomavirus vaccine provided by the present invention contains the above 12 types clearly associated with human cancers, as well as two most common low-risk types: HPV6 and 11, and a suspected carcinogenic type: HPV68. In China, it is expected that the preventive effect of this vaccine on cervical cancer in women will increase to 97.2% or more, while globally, the preventive effect thereof on cervical cancer in women will be expected to increase to 94.1% or more.

The aluminum adjuvant used in the commercially available 9-valent vaccine Gardasil 9 is aluminum hydroxyphosphate sulfate adjuvant (belonging to one of aluminum phosphate adjuvants), and the aluminum adjuvant used in the vaccine provided by the present invention is aluminum hydroxide. The various types of proteins in the vaccines provided by the present invention are prepared by expression after C-terminus truncation, with the parts where positive charges are enriched being removed. The proteins are negatively charged as a whole in pH close to neutral, and are more easily adsorbed by the positively charged aluminum hydroxide adjuvant. Gardasil 9 is prepared by expression of a protein with a full-length sequence. The protein is positively charged as a whole in pH close to neutral, making it less likely to be adsorbed by aluminum hydroxide.

### BRIEF DESCRIPTION OF THE DRAWINGS

Fig. 1: Tm determination results by differential scanning fluorimetry.
Fig. 2: Thermodynamic diagram of Tm values determined by differential scanning fluorimetry.
Fig. 3: Neutralizing antibody titers of vaccines stored at 4°C for 2 weeks.
Fig. 4: Neutralizing antibody titers of vaccines stored at 37°C for 1 week.
Fig. 5: Neutralizing antibody titers of vaccines stored at 37°C for 2 weeks.

### DETAILED DESCRIPTION OF THE EMBODIMENTS

The present invention will be described below through specific embodiments to make the present invention better understood, but it does not constitute a limitation to the present invention.

### Embodiment 1: Preparation of various types of antigens

The embodiment of the present invention adopted various types of L1 proteins as related amino acid sequences of antigens, and preparation methods of the L1 proteins were as follows:

An amino acid sequence of HPV6 L1 protein is as shown in SEQ ID NO: 1, and preparation steps of its antigen proteins are detailed in Embodiment 1, Embodiment 2, Embodiment 10, Embodiment 11, and Embodiment 12 in Patent CN 201410685769.1.

An amino acid sequence of HPV11 L1 protein is as shown in SEQ ID NO: 2, and preparation steps of its antigen proteins are detailed in Embodiment 1, Embodiment 2, Embodiment 10, Embodiment 11, and Embodiment 12 in Patent CN201410672159.8.

An amino acid sequence of HPV16 L1 protein is as shown in SEQ ID NO: 3, and preparation steps of its antigen proteins are detailed in Embodiment 1, Embodiment 2, Embodiment 10, Embodiment 11, and Embodiment 12 in Patent CN201410683185.0.

An amino acid sequence of HPV18 L1 protein is as shown in SEQ ID NO: 4, and preparation steps of its antigen proteins are detailed in Embodiment 1, Embodiment 2, Embodiment 10, Embodiment 11, and Embodiment 12 in Patent CN201410672158.3.

An amino acid sequence of HPV31 L1 protein is as shown in SEQ ID NO: 5, and preparation steps of its antigen proteins are detailed in Embodiment 1, Embodiment 2, Embodiment 10, Embodiment 11, and Embodiment 12 in Patent CN201510490172.6.

An amino acid sequence of HPV33 L1 protein is as shown in SEQ ID NO: 6, and preparation steps of its antigen proteins are detailed in Embodiment 1, Embodiment 2, Embodiment 10, Embodiment 11, and Embodiment 12 in Patent CN201510490177.9.

An amino acid sequence of HPV35 L1 protein is as shown in SEQ ID NO: 7, and preparation steps of its antigen proteins are detailed in Embodiment 1, Embodiment 2, Embodiment 3, and Embodiment 4 in Patent CN202211702926.6.

An amino acid sequence of HPV39 L1 protein is as shown in SEQ ID NO: 8, and preparation steps of its antigen proteins are detailed in Embodiment 1, Embodiment 2, Embodiment 3, and Embodiment 4 in Patent CN202211702935.5.

An amino acid sequence of HPV45 L1 protein is as shown in SEQ ID NO: 9, and preparation steps of its antigen proteins are detailed in Embodiment 1, Embodiment 2, Embodiment 10, Embodiment 11, and Embodiment 12 in Patent CN201510490367.0.

An amino acid sequence of HPV51 L1 protein is as shown in SEQ ID NO: 10, and preparation steps of its antigen proteins are detailed in the full contents of the experimental methods in the specific embodiments of Patent CN202310020457.8.

An amino acid sequence of HPV52 L1 protein is as shown in SEQ ID NO: 11, and preparation steps of its antigen proteins are detailed in Embodiment 1, Embodiment 2, Embodiment 10, Embodiment 11, and Embodiment 12 in Patent CN201510490149.7.

An amino acid sequence of HPV56 L1 protein is as shown in SEQ ID NO: 12, and preparation steps of its antigen proteins are detailed in the full contents of the experimental steps 1, 2, and 3 in the specific embodiments of Patent CN202310019679.8.

An amino acid sequence of HPV58 L1 protein is as shown in SEQ ID NO: 13, and preparation steps of its antigen proteins are detailed in Embodiment 1, Embodiment 2, Embodiment 10, Embodiment 11, and Embodiment 12 in Patent CN201410672161.5.

An amino acid sequence of HPV59 L1 protein is as shown in SEQ ID NO: 14, and preparation steps of its antigen proteins are detailed in Embodiment 1, Embodiment 2, Embodiment 3, and Embodiment 4 in Patent CN202211702927.0.

An amino acid sequence of HPV68 L1 protein is as shown in SEQ ID NO: 15, and preparation steps of its antigen proteins are detailed in Embodiment 1, Embodiment 3, Embodiment 4, and Embodiment 5 in Patent CN202211339029.3. However, the truncated HPV68 L1 protein encoding nucleotide sequence is optimized as shown in SEQ ID NO: 16, and a vector used is pKL30 (Shine-Dalgarno (SD) sequence: 5'-AGGAGGAATTA-3') for constructing recombinant expression.

The antigen sequences of various types can also be found in SEQ ID NO: 1 to SEQ ID NO: 15 in Patent CN202310114443.2, and the overall construction steps can be found in Embodiment 1 and Embodiment 2 in Patent CN202310114443.2.

The preparation methods of the various types of antigens are as follows (as described in the relevant patent applications mentioned above): 1. Resuspension of bacterial cells: a fermentation product (containing 2-5 kg of wet bacterial cells) was taken, and a bacteria breaking buffer was added at a mass ratio of 1:4 to 1:10 between the bacterial cells and a resuspension solution for resuspension. The resuspended bacterial cells were crushed with a high-pressure homogenizer, with a pressure set at 80 MPa. The cells were crushed for three times, and cell slurry obtained after crushing was centrifuged by tubular centrifugation (15760 g, feed flow rate: 200-250 mL/min) to collect the supernatant.

2. Ammonium sulfate precipitation: ammonium sulfate powder was added to the supernatant to a saturation of 25-45%, and slowly stirred at room temperature until completely dissolved. Precipitate was collected by tubular centrifugation (15760g, feed flow rate: 200-250 mL/min). A resolubilization buffer was added to the precipitate at a mass ratio of 1:5 to 1:10 for resuspending until completely dissolved, and centrifugation was carried out again to collect supernatant.

3. Microfiltration clarification: the supernatant from centrifugation was further clarified by two-stage deep filtration, with filter membrane pore sizes of 3.0-6.0 µm and 0.2-0.4 µm.

4. Chromatography: the chromatography process included following three steps: EQ anion exchange chromatography→SQ anion exchange chromatography→gel filtration chromatography. The operation is briefly described as follows:
(1) EQ anion exchange chromatography: After column equilibration, a sample collected after deep filtration was load, a wash after loading completion was performed, and an EQ ion exchange flow-through liquid with OD280 greater than 50 mAU was collected.
(2) SQ anion exchange chromatography: After column equilibration, the EQ ion exchange flow-through liquid from the step (1) was loaded; a wash after loading completion was performed; and an elution after the wash ends was performed, and a target protein absorption peak with OD280 not less than 40 mAU was collected during elution.
(3) Gel filtration chromatography: After a chromatographic column was equilibrated until a baseline was stable, a target protein sample collected from SQ anion exchange chromatography elution was loaded, and an HPV L1 protein absorption peak with OD280 greater than 50 mAU was collected.

5. VLP assembly, liquid exchange and stock solution preservation: the purified HPV L1 protein was self-assembled *in vitro* to form VLPs, the VLPs were enabled to be further subjected to column chromatography for liquid exchange, sterilization and filtration to prepare bulk protein solutions, and the bulk protein solutions were stored at -80°C for later use. A minimum storage concentration of all the types of bulk protein solutions is 1.56 mg/mL, and a pH range of buffers used is 4.3-5.8.

### Embodiment 2: Study on buffer systems of the preparation

The main purpose of studying buffer systems of the preparation was to determine a pH range and an optimal concentration of sodium chloride. Two research methods were selected: one was to use a differential scanning fluorimetry to determine the Tm value of each type of L1-VLP antigen under different preparation prescriptions (different in pH and salt concentration). The higher the Tm value, the more stable the antigen under this prescription. Based on the determination results of various types under the prescriptions, a parameter range favorable for antigen stability was selected. The other method is to prepare a 15-valent HPV vaccine under different preparation prescriptions (different in pH and salt concentration); and after the vaccine was placed at 37°C for 1-2 weeks, an appropriate dose (such as 1/20× for humans) was selected to immunize animals and a neutralizing antibody level was determined. The neutralizing antibody level after immunization with the vaccine placed in a buffer system that was conducive to antigen stability should be higher, thereby determining the appropriate preparation pH range and sodium chloride concentration.

The L1-VLP stock solution of each type of the vaccine provided by invention was formulated as 20 mM acetic acid-sodium acetate, 500-700 mM sodium chloride, and 0.02% polysorbate 80, with pH of 4.8-5.3. During the vaccine preparation process, histidine was introduced to adjust the pH to neutral to increase body compliance. A buffer system of the preparation was formulated as: 10 mM histidine, 10 mM acetic acid-sodium acetate, 327 mM sodium chloride and 0.01% polysorbate 80.

A total of 20 buffer systems of the preparation were set up in this study, including 15 systems containing histidine (pH 5.3, 5.6, 5.9, 6.2, 6.5, sodium chloride 154, 327, 500 mM) and 5 systems without histidine (pH 4.4, 4.7, 5.0, 5.3, 5.6, sodium chloride 327 mM). See Table 1 for details.

**Table 1 List of buffer systems of the preparation**

| **Prescription No.** | **Acetic acid-Sodium acetate (mM)** | **Histidine (mM)** | **Sodium chloride (mM)** | **Polysorbate 80 (%)** | **pH** |
|---|---|---|---|---|---|
| 1 | 10 | 10 | 154 | 0.01 | 5.3 |
| 2 | 10 | 10 | 154 | 0.01 | 5.6 |
| 3 | 10 | 10 | 154 | 0.01 | 5.9 |
| 4 | 10 | 10 | 154 | 0.01 | 6.2 |
| 5 | 10 | 10 | 154 | 0.01 | 6.5 |
| 6 | 10 | 10 | 327 | 0.01 | 5.3 |
| 7 | 10 | 10 | 327 | 0.01 | 5.6 |
| 8 | 10 | 10 | 327 | 0.01 | 5.9 |
| 9 | 10 | 10 | 327 | 0.01 | 6.2 |
| 10 | 10 | 10 | 327 | 0.01 | 6.5 |
| 11 | 10 | 10 | 500 | 0.01 | 5.3 |
| 12 | 10 | 10 | 500 | 0.01 | 5.6 |
| 13 | 10 | 10 | 500 | 0.01 | 5.9 |
| 14 | 10 | 10 | 500 | 0.01 | 6.2 |
| 15 | 10 | 10 | 500 | 0.01 | 6.5 |
| 16 | 10 | - | 327 | 0.01 | 4.4 |
| 17 | 10 | - | 327 | 0.01 | 4.7 |
| 18 | 10 | - | 327 | 0.01 | 5.0 |
| 19 | 10 | - | 327 | 0.01 | 5.3 |
| 20 | 10 | - | 327 | 0.01 | 5.6 |

### ① Differential scanning fluorimetry (DSF)

The Tm values of fifteen types of HPV L1-VLP were determined by a DSF under 20 preparation prescriptions one by one.

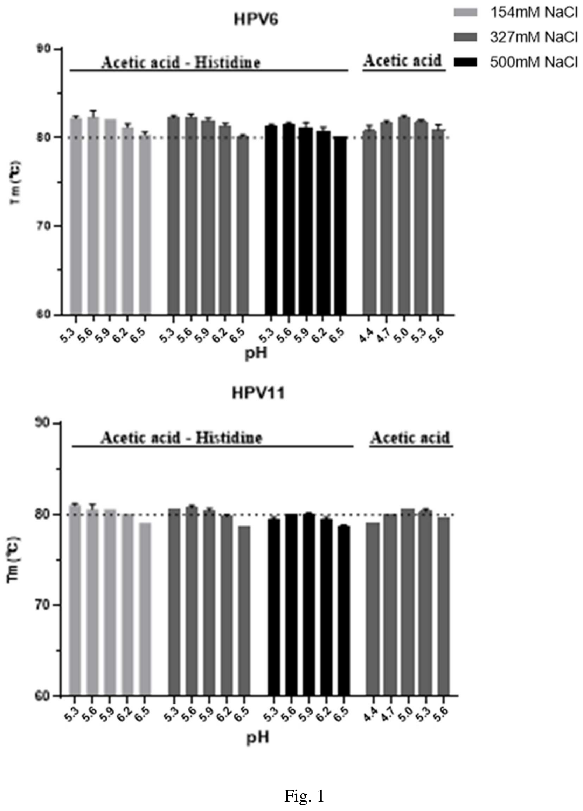

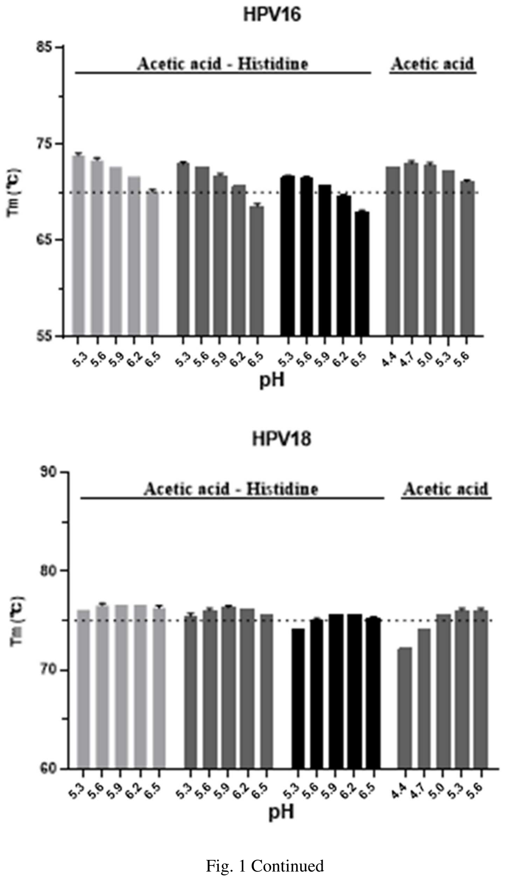

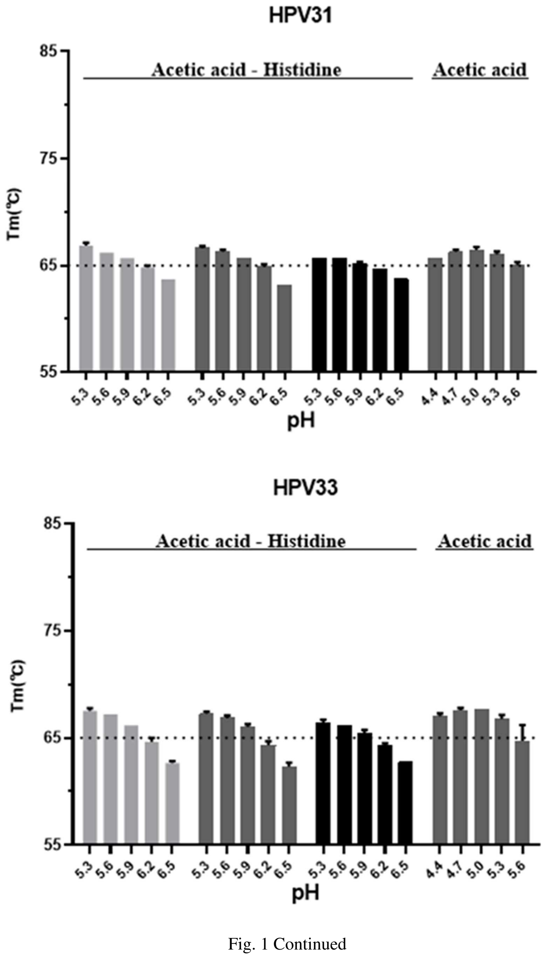

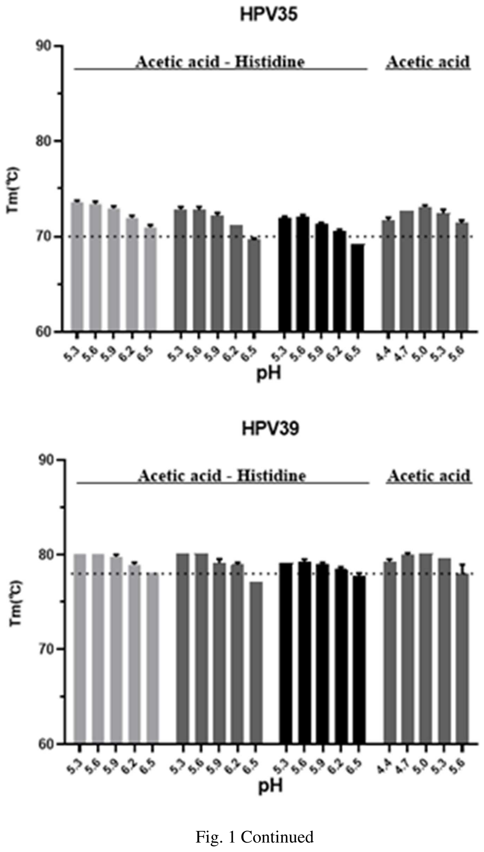

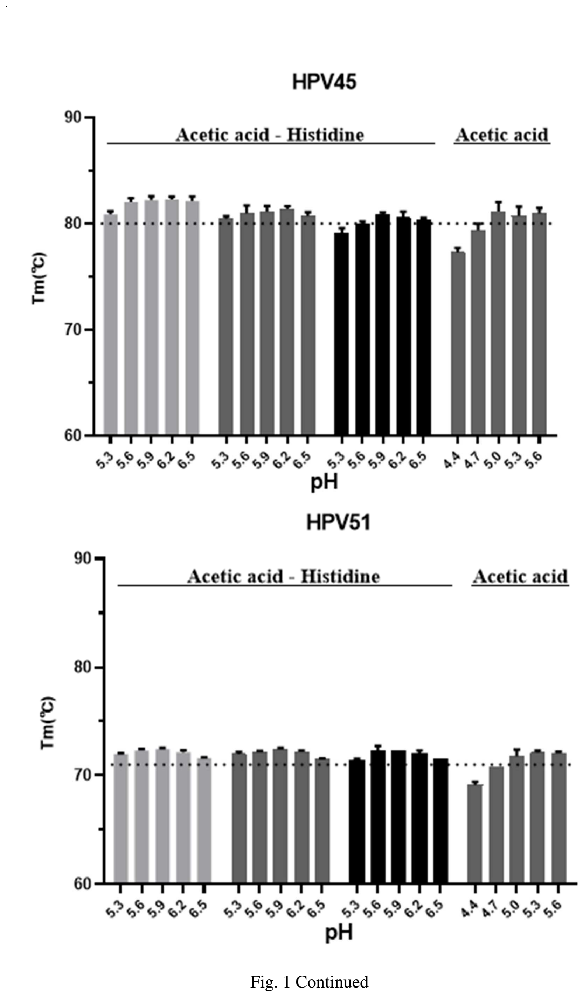

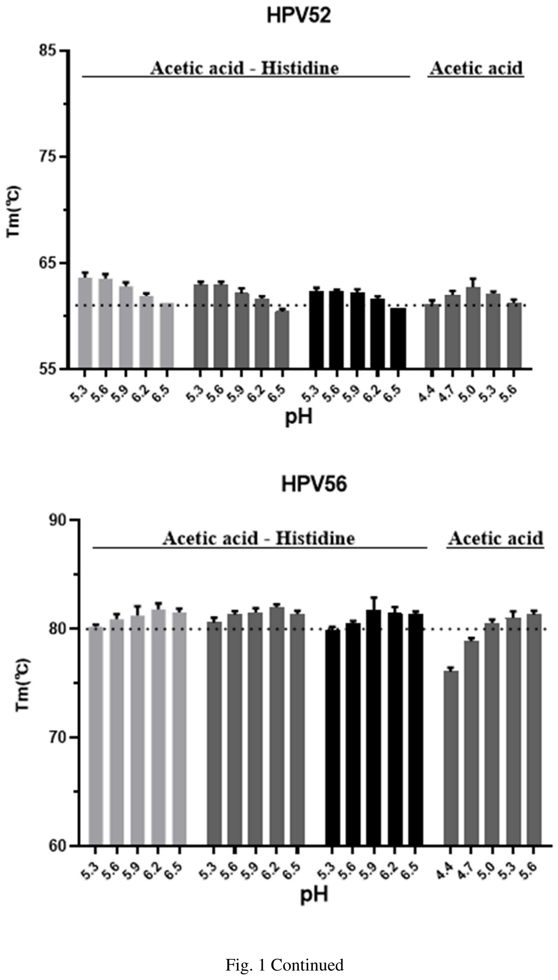

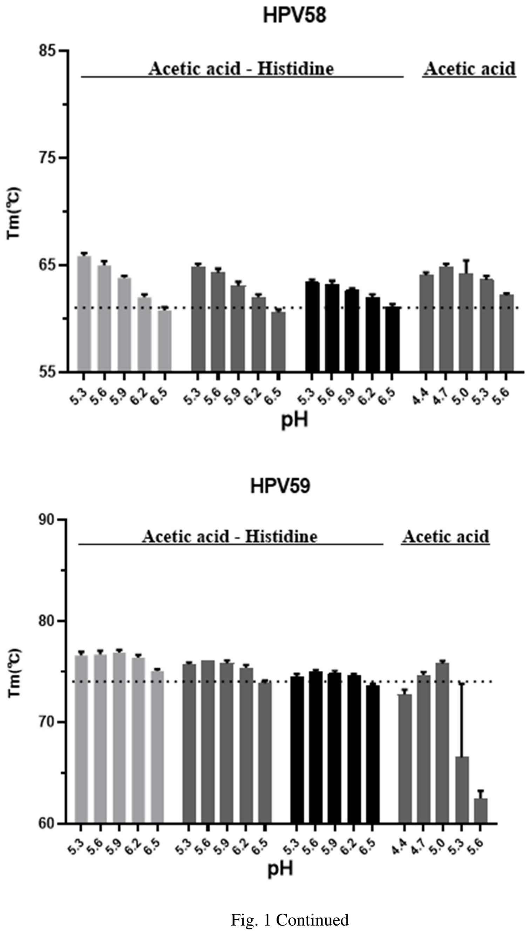

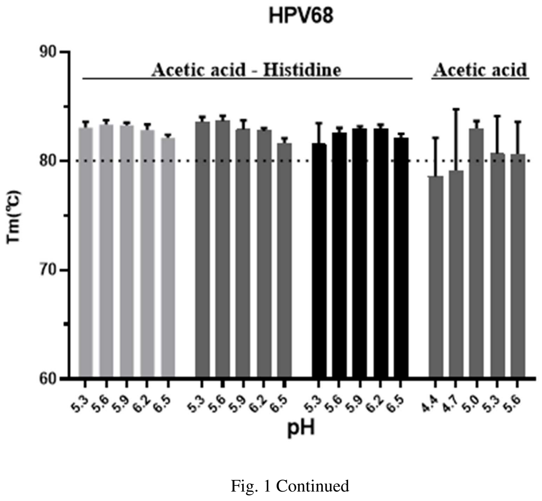

The results show (as shown in Fig. 1) that the Tm values of HPV6 and 11, both of which belong to the α10 genus in virus taxonomy, are relatively high, the Tm values of HPV16, 31, 33, 35, 52, and 58, all of which belong to the α9 genus, are relatively low or medium, the Tm values of HPV18, 39, 45, 59, and 68, all of which belong to the α7 genus, are relatively high or medium, and HPV51 and HPV56 respectively belong to α5 genus and α6 genus. The results show that there is a certain correlation between the Tm value and the evolutionary relationship of each type of virus.

In buffers containing histidine and acetic acid, the effects of salt concentrations and pH on Tm values can be divided into three categories: ① for HPV6, HPV11, HPV16, HPV31, HPV33, HPV35, HPV39, HPV52, and HPV58, in the case where these nine types exhibit low salt concentrations and low pH, Tm values are high; ② for HPV18, HPV45, and HPV59, in the case where these three types exhibit low salt concentrations and medium pH, Tm values are high; and ③ for HPV51, HPV56, and HPV68, in the case where these three types exhibit insensitivity to salt concentration and medium pH, Tm values are high.

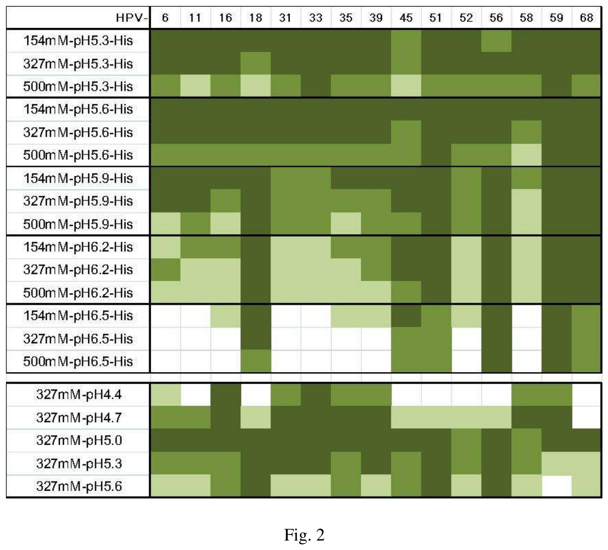

In order to further analyze the preparation prescription of the 15-valent HPV vaccine, the Tm values obtained by the DSF were plotted as a thermodynamic diagram, and the darker the color, the higher the Tm value (see Fig. 2). Under the same salt concentration (327 mM) and the same pH (5.3 or 5.6), the Tm value of an acetate buffer containing histidine is significantly higher than the Tm value of an acetate buffer without histidine. Therefore, the scope of the preparation prescription is narrowed down to the acetate buffer containing histidine. In the acetate buffer containing histidine, pH 5.3-6.2 is significantly better than pH 6.5, so that the scope of the preparation prescription is initially set at pH 5.3-6.2. Under the condition of pH 5.3-6.2, the salt concentrations of 154 mM and 327 mM are obviously better than 500 mM.

According to the analysis results of antigen Tm values under all the preparation prescriptions, the preparation prescription stability range is determined as follows: pH: 5.3-6.2 and NaCl concentration: 154-327 mM.

### ②Neutralization antibody method

The thirteen types of L1-VLP stock solutions in the vaccine of this embodiment were formulated as 20 mM acetic acid-sodium acetate, 500 mM sodium chloride, and 0.02% polysorbate 80, with pH of 5.0. On this basis, the HPV31 stock solution was optimally formulated with pH of 4.8 and salt concentration of 700 mM. The optimal pH of HPV56 was 5.3. First, a 15-valent HPV vaccine with or without histidine was prepared, and the prepared vaccine samples were subpackaged (with 15 vials containing histidine and 5 vials without histidine). After centrifugation, supernatant was removed, and the precipitate was resuspended with the same volume of 20 preparation prescription solutions. After fully mixing well, the vaccine sample in each vial was evenly divided into three parts, which were respectively put into 3 vials and then sealed. For each preparation prescription, one vial was placed at 4°C as a control. The other two vials were placed in an incubator at 37°C, one vial was taken out on the 7^{th} day and temporarily stored at 4°C, and the other vial was taken out on the 14^{th} day and temporarily stored at 4°C.

0.3mL of the vaccine sample was taken after placement, and diluted 4 times to 1.2mL with a corresponding preparation buffer for mouse immunization. Blood was collected at 4 weeks after immunization, with serum being separated, and neutralizing antibody titers of 15 types of HPV were determined by a neutralizing antibody detection method based on HPV pseudovirus.

The neutralizing antibody titers were calculated by a Reed-Muench method, and the geometric mean titers of neutralizing antibodies were calculated and compared between groups (Mann-Whitney Test) with Graphpad Prism software, so as to evaluate the influence of different preparation prescriptions on vaccine stability and determine the appropriate range of preparation prescription parameters.

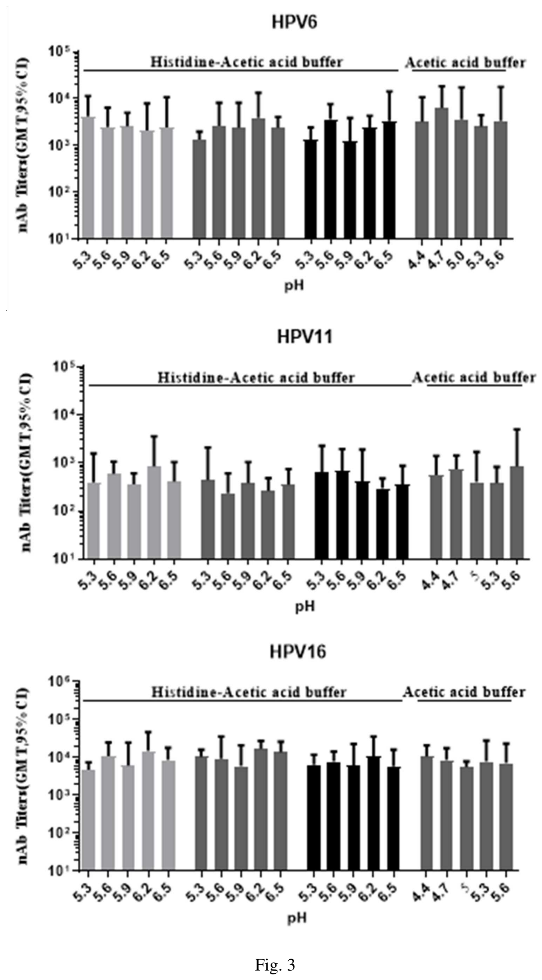

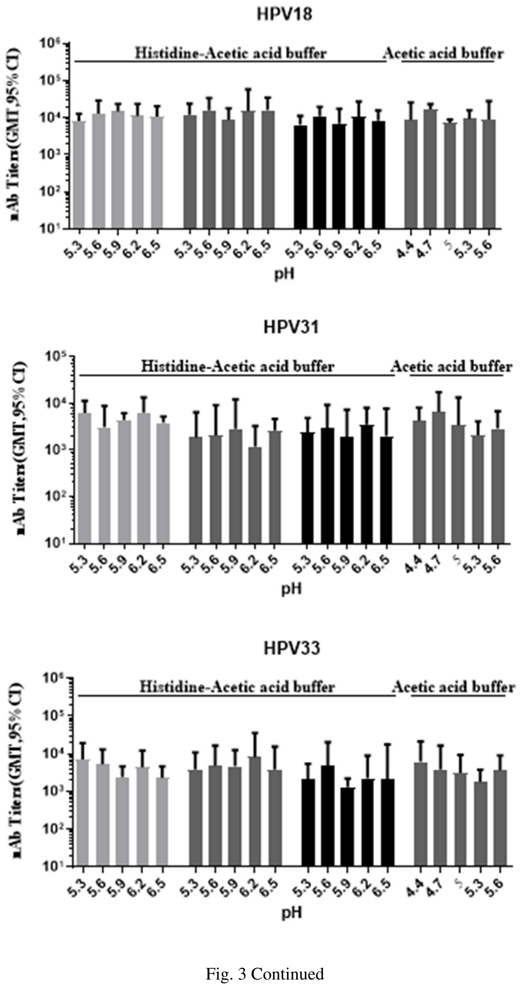

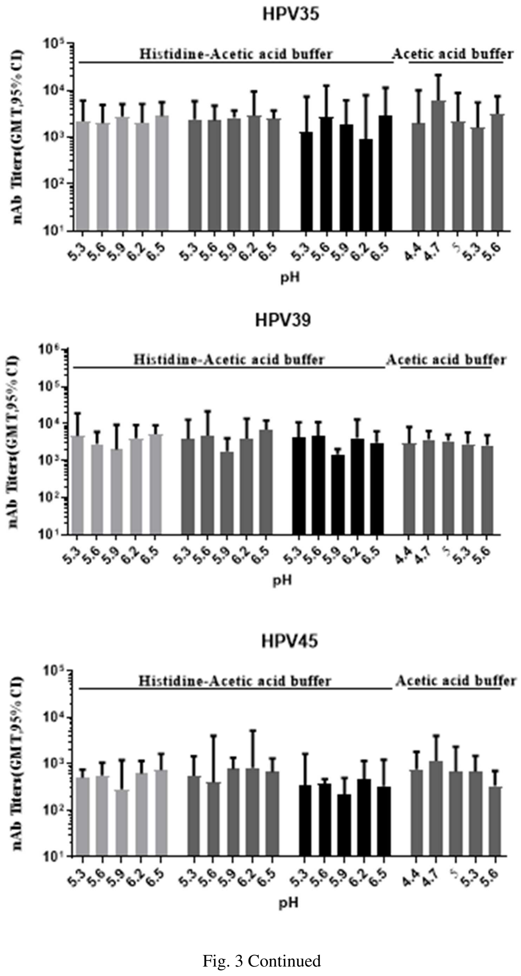

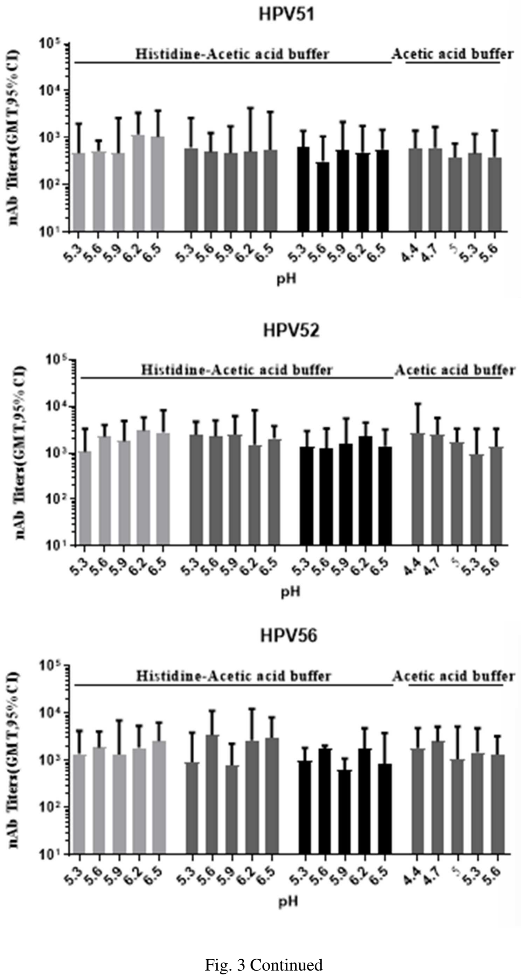

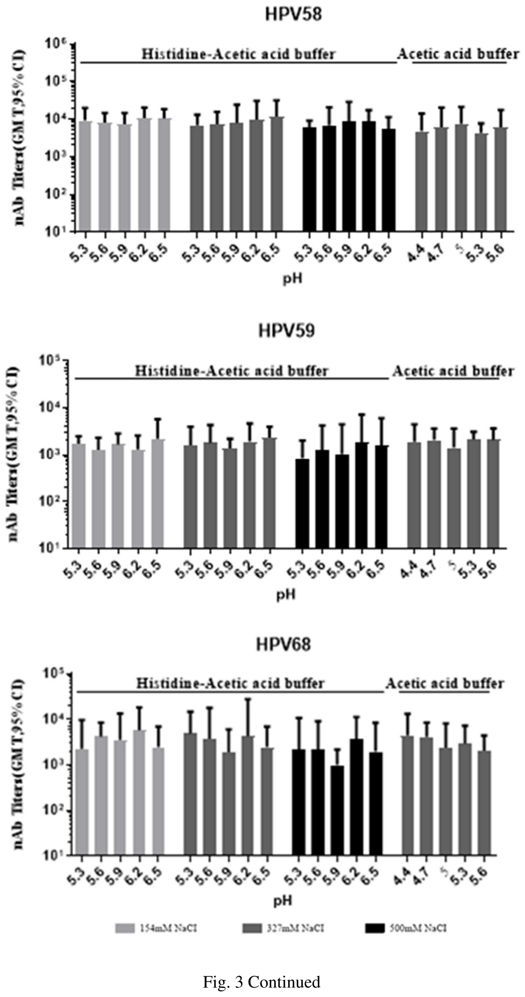

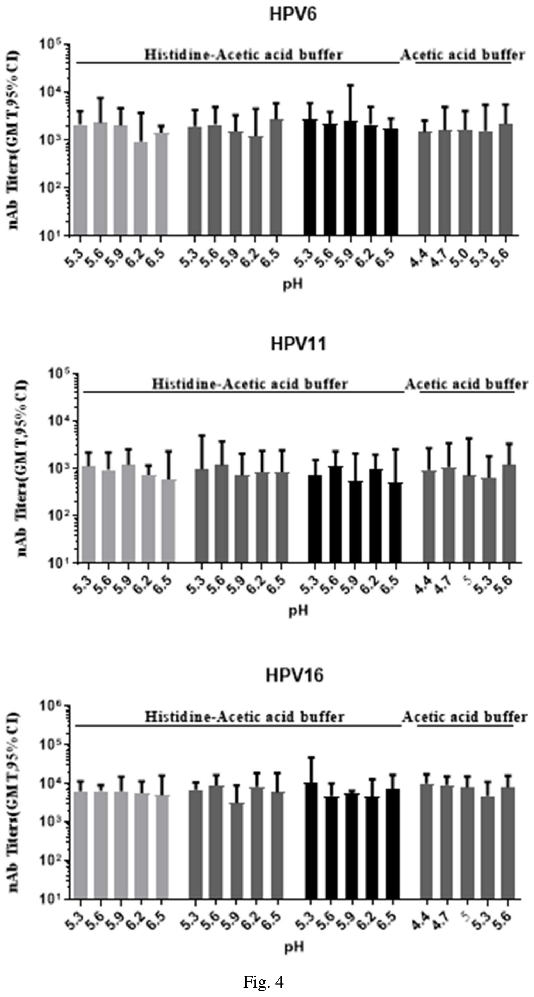

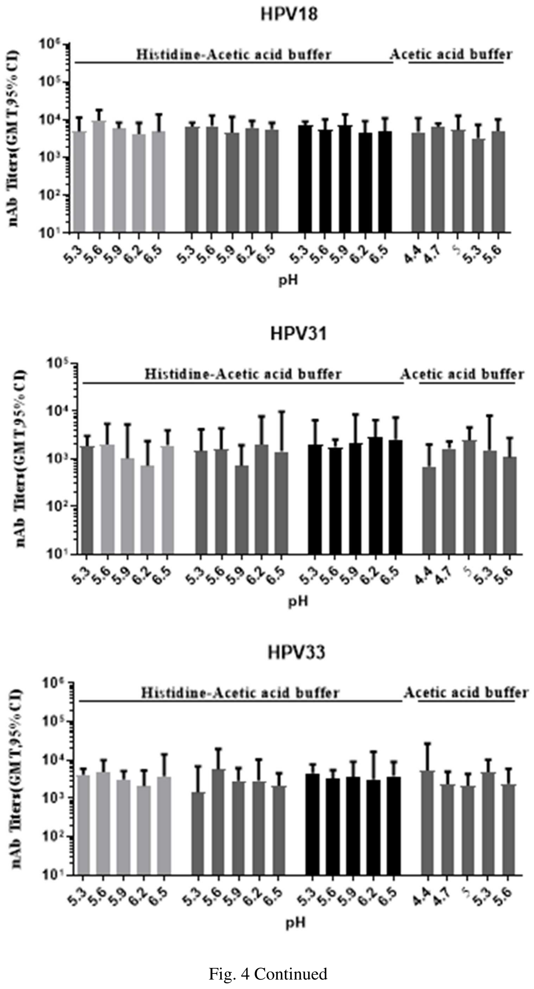

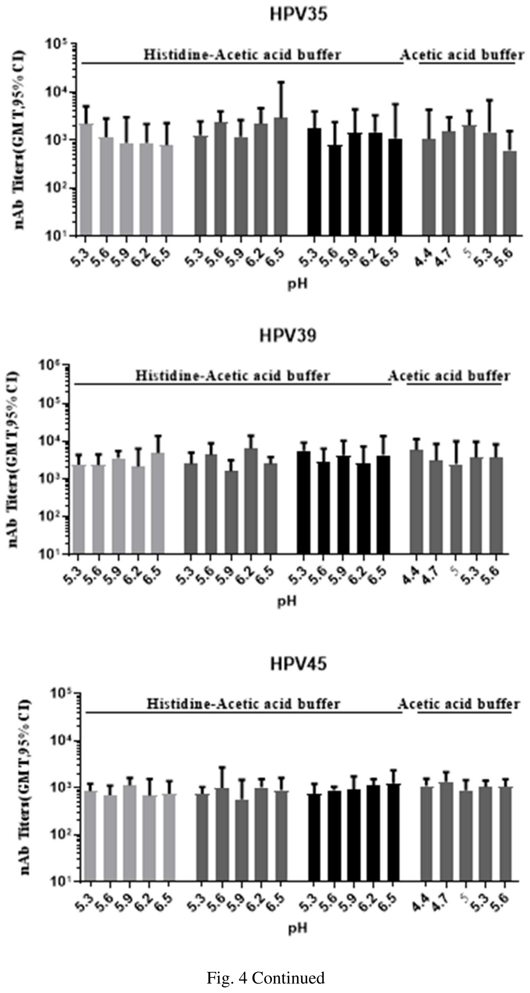

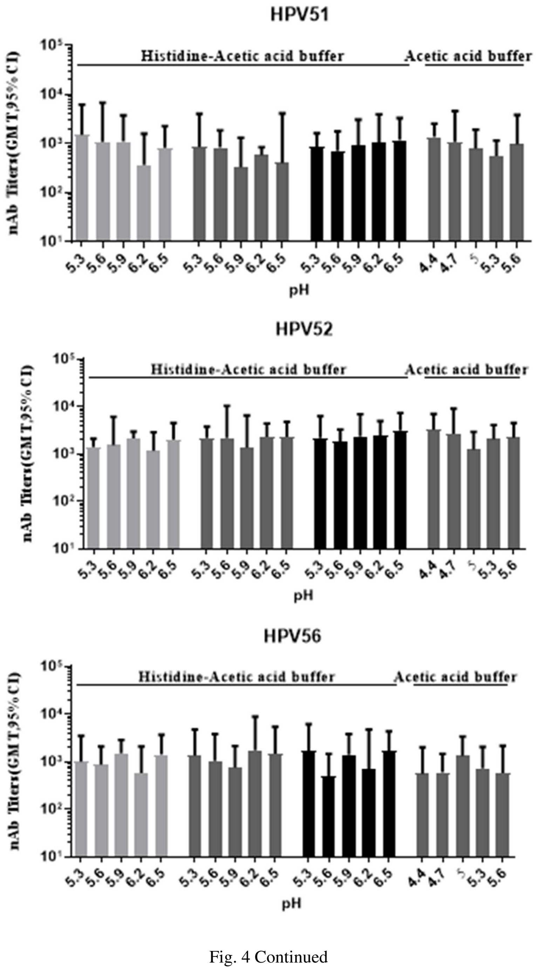

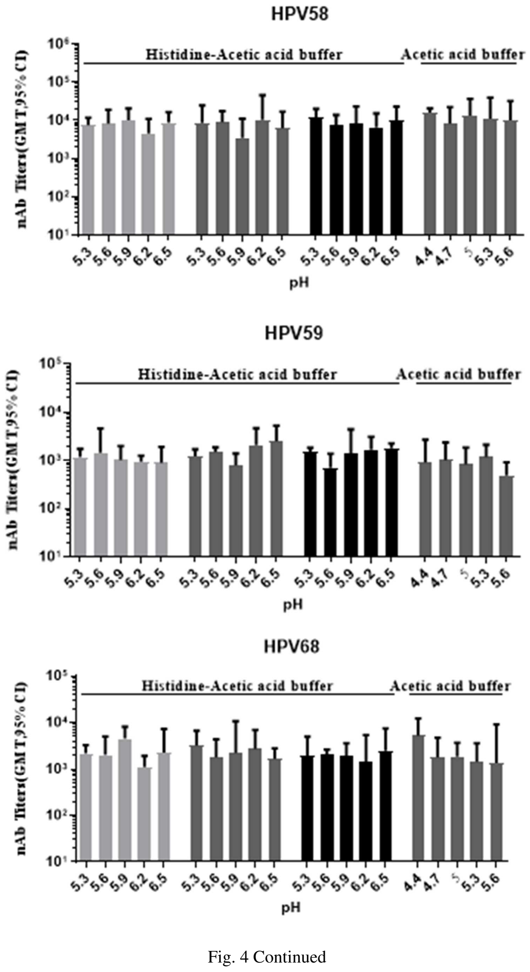

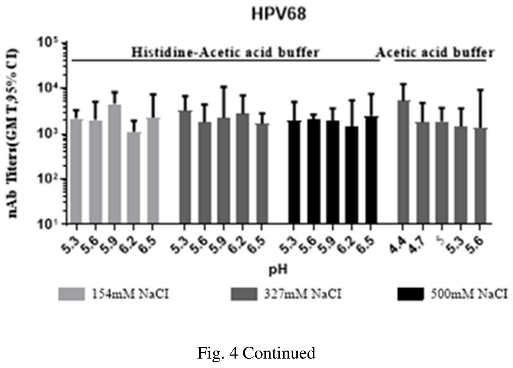

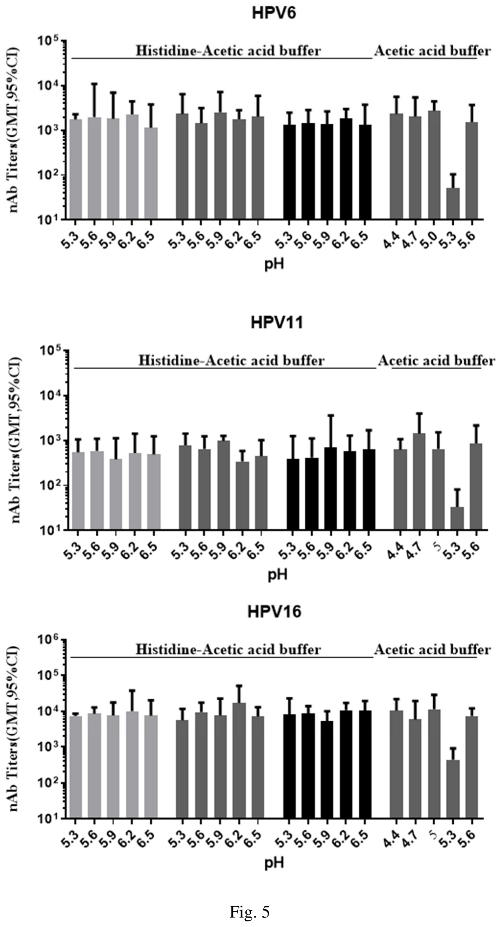

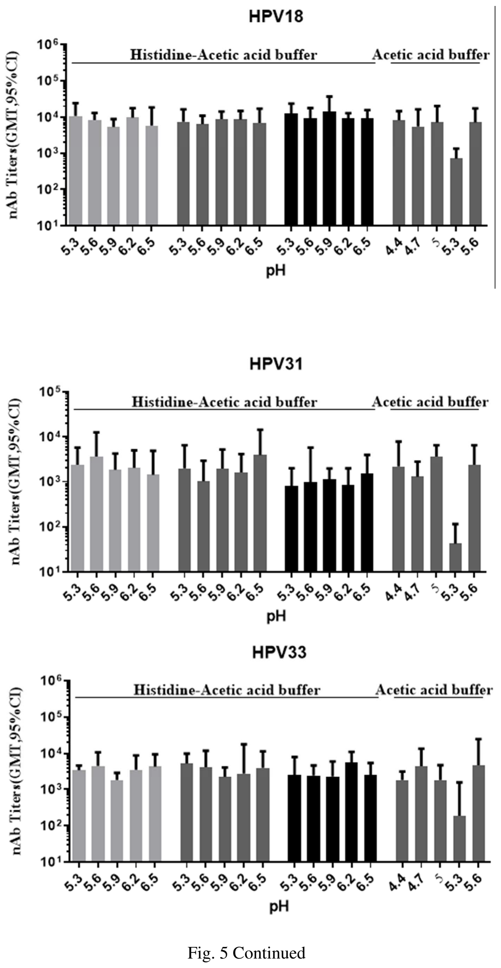

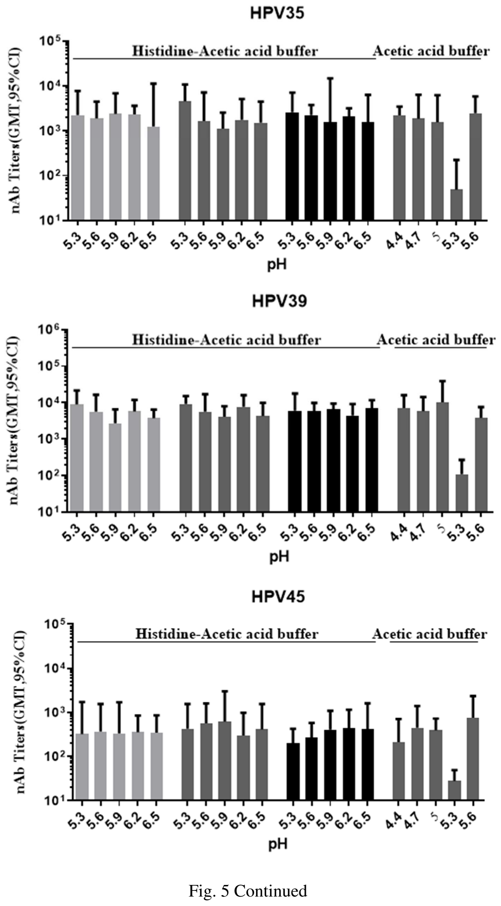

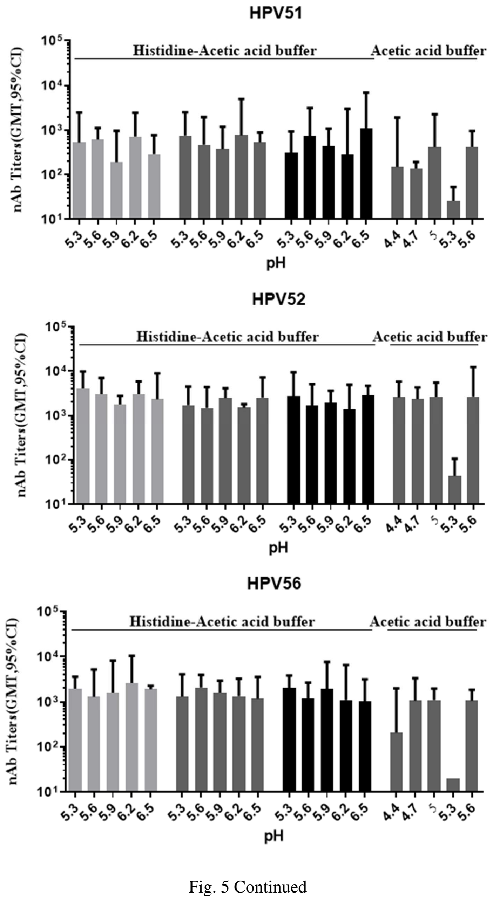

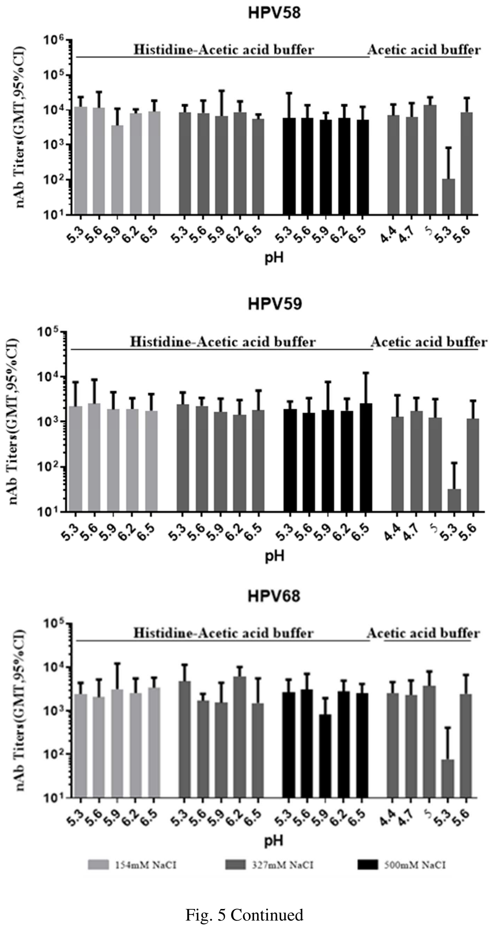

The results of neutralizing antibody detection are shown in Fig. 3, Fig. 4 and Fig. 5. The 15-valent HPV vaccines prepared according to 20 preparation prescriptions were respectively placed at 4°C for 2 weeks, and at 37°C for 1 week and 2 weeks, and then applied for immunization of mice at 1/20× of the human dose. Blood was collected at 4 weeks after immunization, the neutralizing antibody titers measured did not show significant differences under different preparation prescriptions, and there was no regular trend, indicating that the buffer system pH (histidine-sodium acetate system 5.3-6.5, acetic acid-sodium acetate system 4.4-5.6) and sodium chloride concentration (histidine-sodium acetate system 154, 327, 500 mM) had little effect on the stability of vaccine immunogenicity within the research scope. In addition, the neutralizing antibody titer measured after an accelerated reaction at 37°C was not significantly different from the neutralizing antibody level measured after placement at 4°C for two weeks (without an accelerated reaction), indicating that the stability of each type of vaccine antigen in the 20 preparation prescriptions was very good.

From the perspective of human acceptance, the pH of the vaccine preparation tends to be neutral (pH 7.0) and the salt concentration thereof is close to 0.9% (physiological saline concentration), which is less irritating to the human body. Therefore, it is more appropriate to choose a histidine-sodium acetate buffer system (pH 5.3-6.5) with a pH closer to neutral as a buffer component of the 15-valent HPV vaccine preparation. Since the salt concentrations of various types of bulk protein solutions (500, 700 mM) are high, and the initial salt concentration of the aluminum hydroxide adjuvant is 0.9% (154 mM), during the preparation of the vaccine preparation, the sodium chloride salt concentration is set to 327 mM for facilitating preparation process operation. Moreover, the results of the stock solution preparation process and prescription research show that high salt concentration is more conducive to the antigen maintaining a uniform 72-pentamer L1-VLP conformation, which is beneficial to the stability of the antigen structure.

### Embodiment 3: Study on antigen dosage ratio and aluminum adjuvant dosage

Based on the various types of VLP antigen proteins obtained in Embodiment 1, 15-valent HPV vaccines with different antigen dosage ratios and aluminum adjuvant dosages (see Table 2) were prepared according to the subsequent preparation process, and BALB/c mice were immunized to investigate the immunogenicity. Specifically, it included: setting a monovalent antigen immunization group to investigate immune interference. Taking Gardasil9 as a control vaccine, different aluminum adjuvant dosage groups were set to determine the adjuvant dosages in the vaccines, and different antigen dosage ratios were set to determine the dosage ratios of various types of antigens. Animal grouping information and settings of antigen dosage ratios and aluminum adjuvant dosages are shown in Table 2. The vaccination program adopted 0 and 4 weeks of immunization, and the neutralizing antibody titers were measured at 4 weeks after the first immunization and at 4 weeks after the second immunization. Comparison between groups was carried out to determine the dosage ratios of various types of antigens and the dosages of aluminum adjuvants in the vaccines.

**Table 2 Animal grouping table for study on antigen dosage ratio and aluminum adjuvant dosage**

| **Group** | **Immune sample** | **Antigen dose for each type of HPV/µg** | | | | | | | | | | | | | | | **Aluminium adjuvant /µg** | **Number of animals** |
|---|---|---|---|---|---|---|---|---|---|---|---|---|---|---|---|---|---|---|
| | | **6** | **11** | **16** | **18** | **31** | **33** | **45** | **52** | **58** | **35** | **39** | **51** | **56** | **59** | **68** | | |
| Group 1 | Monovalent 1/20 | 1. 5 | - | - | - | - | - | - | - | - | - | - | - | - | - | - | 37.5 | 10 |
| Group 2 | Monovalent 1/20 | - | 2 | - | - | - | - | - | - | - | - | - | - | - | - | - | 37.5 | 10 |
| Group 3 | Monovalent 1/20 | - | - | 3 | - | - | - | - | - | - | - | - | - | - | - | - | 37.5 | 10 |
| Group 4 | Monovalent 1/20 | - | - | - | 2 | - | - | - | - | - | - | - | - | - | - | - | 37.5 | 10 |
| Group 5 | Monovalent 1/20 | - | - | - | - | 1 | - | - | - | - | - | - | - | - | - | - | 37.5 | 10 |
| Group 6 | Monovalent 1/20 | - | - | - | - | - | 1 | - | - | - | - | - | - | - | - | - | 37.5 | 10 |
| Group 7 | Monovalent 1/20 | - | - | - | - | - | - | 1 | - | - | - | - | - | - | - | - | 37.5 | 10 |
| Group 8 | Monovalent 1/20 | - | - | - | - | - | - | - | 1 | - | - | - | - | - | - | - | 37.5 | 10 |
| Group 9 | Monovalent 1/20 | - | - | - | - | - | - | - | - | 1 | - | - | - | - | - | - | 37.5 | 10 |
| Group 10 | Monovalent 1/20 | - | - | - | - | - | - | - | - | - | 1 | - | - | - | - | - | 37.5 | 10 |
| Group 11 | Monovalent 1/20 | - | - | - | - | - | - | - | - | - | - | 1 | - | - | - | - | 37.5 | 10 |
| Group 12 | Monovalent 1/20 | - | - | - | - | - | - | - | - | - | - | - | 1 | - | - | - | 37.5 | 10 |
| Group 13 | Monovalent 1/20 | - | - | - | - | - | - | - | - | - | - | - | - | 1 | - | - | 37.5 | 10 |
| Group 14 | Monovalent 1/20 | - | - | - | - | - | - | - | - | - | - | - | - | - | 1 | - | 37.5 | 10 |
| Group 15 | Monovalent 1/20 | - | - | - | - | - | - | - | - | - | - | - | - | - | - | 1 | 37.5 | 10 |
| **Group 16** | **15-valent 1/20** | **1. 5** | **2** | **3** | **2** | **1** | **1** | **1** | **1** | **1** | **1** | **1** | **1** | **1** | **1** | **1** | **37.5** | **10** |
| Group 17 | 15-valent 1/20 | 1. 5 | 2 | 3 | 2 | 1 | 1 | 1 | 1 | 1 | 1 | 1 | 1 | 1 | 1 | 1 | 0 | 10 |
| Group 18 | 15-valent 1/20 | 1. 5 | 2 | 3 | 2 | 1 | 1 | 1 | 1 | 1 | 1 | 1 | 1 | 1 | 1 | 1 | **25** | 10 |
| Group 19 | 15-valent 1/20 | 1. 5 | 2 | 3 | 2 | 1 | 1 | 1 | 1 | 1 | 1 | 1 | 1 | 1 | 1 | 1 | **50** | 10 |
| Group 20 | 15-valent 1/20 | 1. 5 | 2 | **3.5** | **2.5** | 1 | **1** | 1 | 1 | 1 | 1 | 1 | 1 | 1 | 1 | 1 | 37.5 | 10 |
| Group 21 | 15-valent 1/20 | 1. 5 | 2 | **3.5** | **2.5** | **1.5** | **1. 5** | **1.5** | **1.5** | **1.5** | 1 | 1 | 1 | 1 | 1 | 1 | 37.5 | 10 |
| Group 22 | Gardasil 9 1/20 | 1. 5 | 2 | 3 | 2 | 1 | 1 | 1 | 1 | 1 | - | - | - | - | - | - | 25 | 10 |
| Group 23 | Aluminum adjuvant control | - | - | - | - | - | - | - | - | - | - | - | - | - | - | - | 37.5 | 5 |

| | | | | | | | | | | | | | | | | | | |
|---|---|---|---|---|---|---|---|---|---|---|---|---|---|---|---|---|---|---|
| Note: Intramuscular injection was used, with 100 µl per BALB/c mouse. Note: Group 1 to Group 15 are antigens of single types (i.e., HPV6, 11, 16, 18, 31, 33, 35, 39, 45, 51, 52, 56, 58, 59, 68) adsorbed with aluminum adjuvants, and 10 mice were immunized. Group 16 to Group 19 are 15-valent antigens of the same type with the same dosage adsorbed with different aluminum adjuvants, and 10 mice were immunized. Group 20 to Group 21 are different doses of antigens of various types adsorbed with the same dose of adjuvant, and 10 mice were immunized. Group 22 is the antigen and adjuvant dose of Merck's commercially available product Gardasil 9-valent vaccine, and 10 mice were immunized as a comparative study. Group 23 is an aluminum adjuvant control group, and 5 mice were immunized. | | | | | | | | | | | | | | | | | | |

See Table 3 for the geometric mean of neutralizing antibody detection at 4 weeks after the first immunization and at 4 weeks after the second immunization. The results show that:

### (1) Immunization interference

Mice were immunized with the same dosage of aluminum adjuvant in the forms of a monovalent antigen and a 15-valent antigen (with the same type of antigen having the same dosage), and the difference in immune response levels of the two groups was compared to investigate immunization interference. The results showed that the neutralizing antibody titers of all types of antigens available for immunization in a monovalent form were significantly higher than that of the antigen in a 15-valent HPV vaccine form, and the titers were all greater than that of the corresponding type of control vaccine (Gardasil9).

### (2) Aluminum adjuvant dosage

The fixed antigen dose was 1/20× of the human dose, and four aluminum adjuvant doses were set: 37.5 (1/20× of the 9-valent vaccine), 0, 25, and 50 µg. Mice were immunized (the number of mice in each group was n=10) to investigate the differences between aluminum adjuvant doses, and comparison with the control vaccine (Gardasil9) was carried out. The results showed that the neutralizing antibody level in the aluminum adjuvant-free group was significantly lower than that in the aluminum adjuvant-containing group, indicating that it was necessary to add an aluminum adjuvant to the vaccine, and the aluminum adjuvant had obvious effect on improving the immune response level of an antigen. There was no significant difference in most types among the three doses in the aluminum adjuvant-containing group. Compared with the control vaccine, the introduction of an aluminum adjuvant did not produce obvious superiority, indicating that 25 µg (corresponding to 500 µg for human dose) of an aluminum adjuvant could reach or approach immune saturation, and its immune response level was equivalent to that of the control vaccine.

### (3) Antigen dosage ratio

The initial antigen dosage of the 15-valent HPV vaccine was determined as addition of 20 µg of each of the six new types of antigens on the basis of the 9-valent vaccine (i.e., containing HPV6, 11, 16, 18, 31, 33, 35, 39, 45, 51, 52, 56, 58, 59, and 68 antigens, which were 30, 40, 60, 40, 20, 20, 20, 20, 20, 20, 20, 20, 20, 20, and 20 µg, respectively). On this basis, the HPV16 and 18 antigens were adjusted, each of which was increased by 10 µg, or the HPV16, 18, 31, 33, 45, 52, and 58 antigens were adjusted, each of which was increased by 10 µg, to investigate whether increasing the dosages of these types of antigens can significantly increase the immune response levels of these types, thus ensuring that the vaccine efficacy was not lower than that of the marketed vaccine Gardasil9. The results (Table 3) showed that compared with the initial antigen dosage, increasing the dosage of each of these types of antigens by 10 µg did not produce a significant immune enhancement effect, indicating that the initial dosage ratio of antigens in mice has reached immune saturation and there is no need to increase the dosages of these types of antigens.

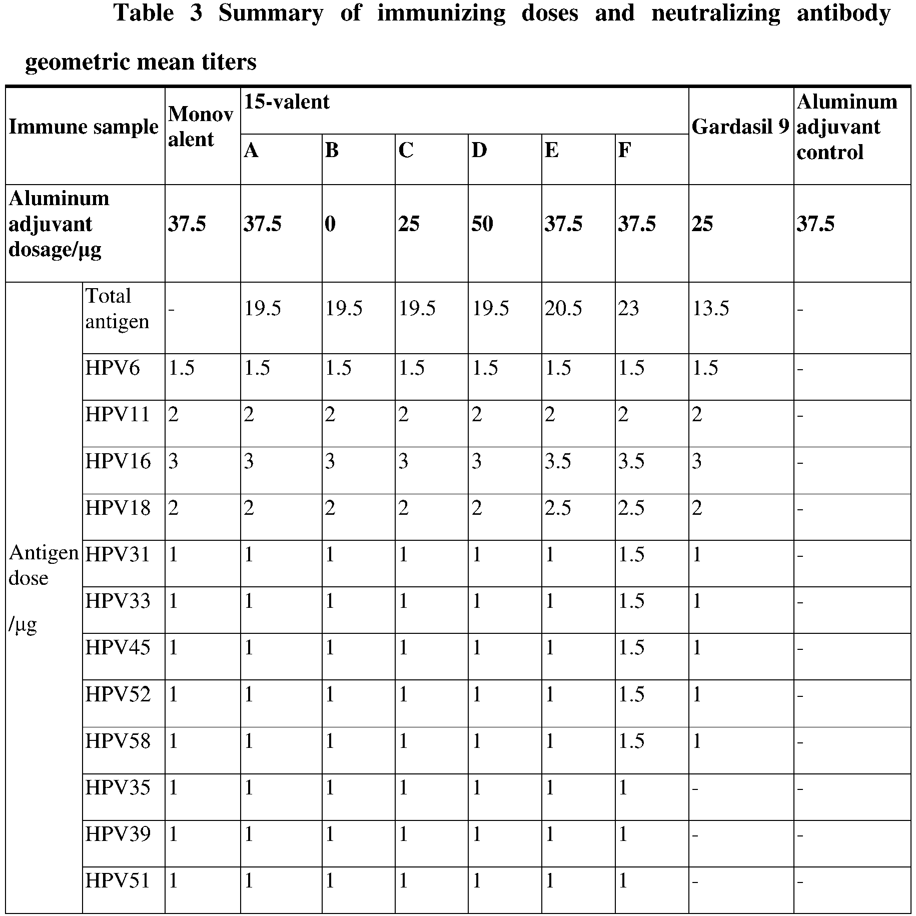

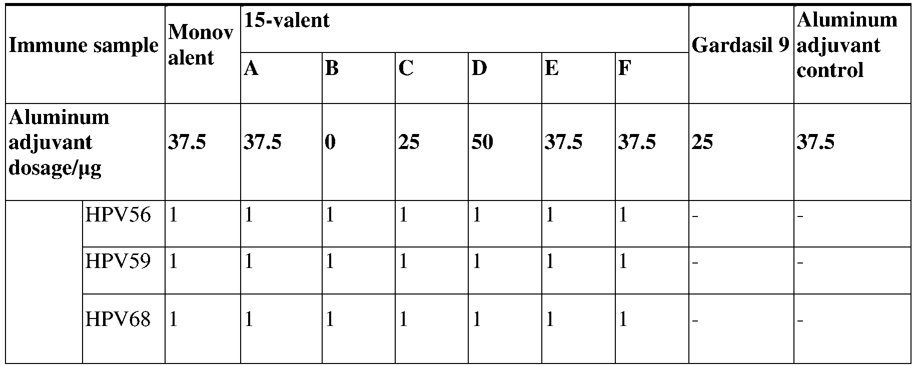

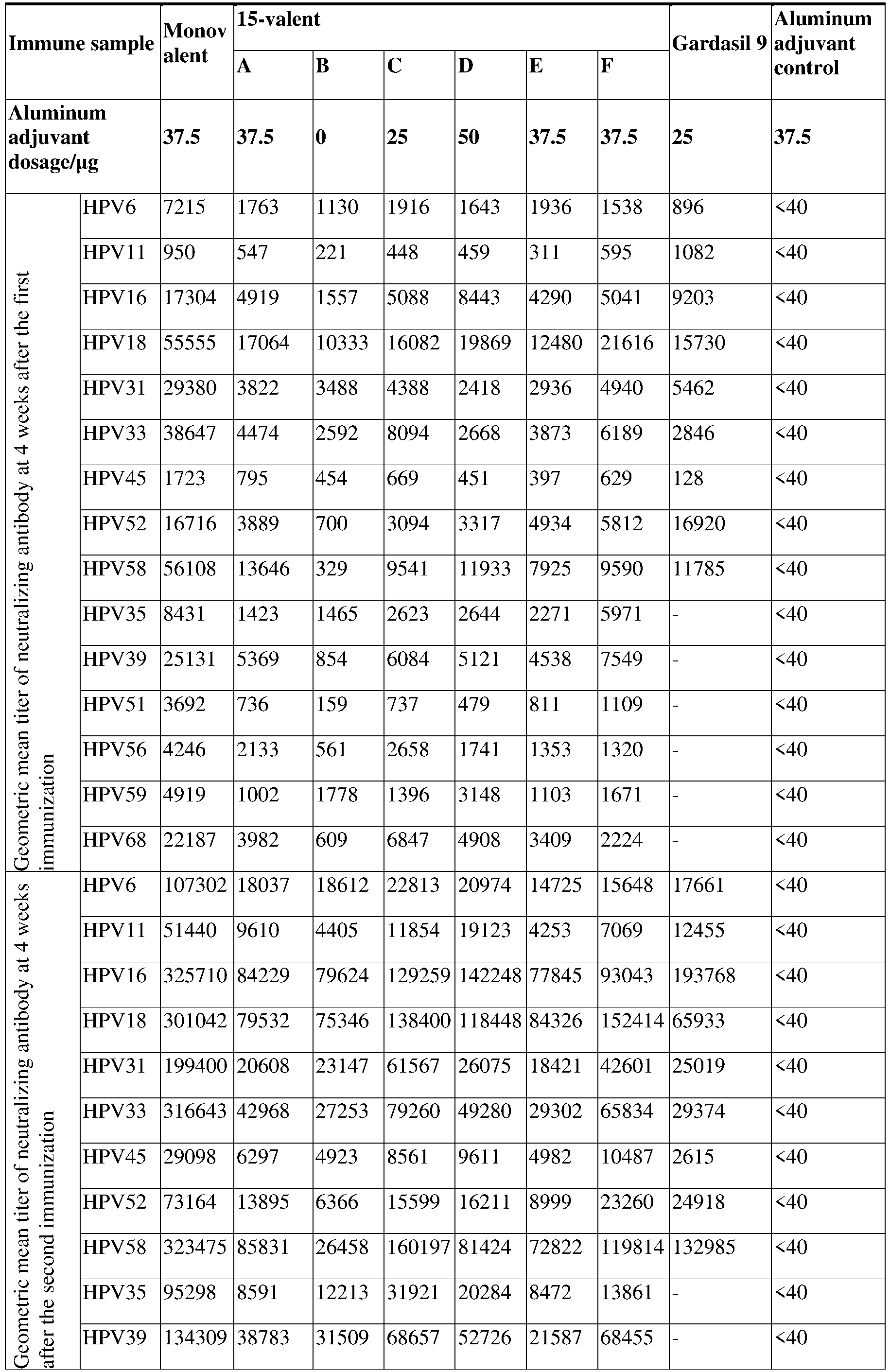

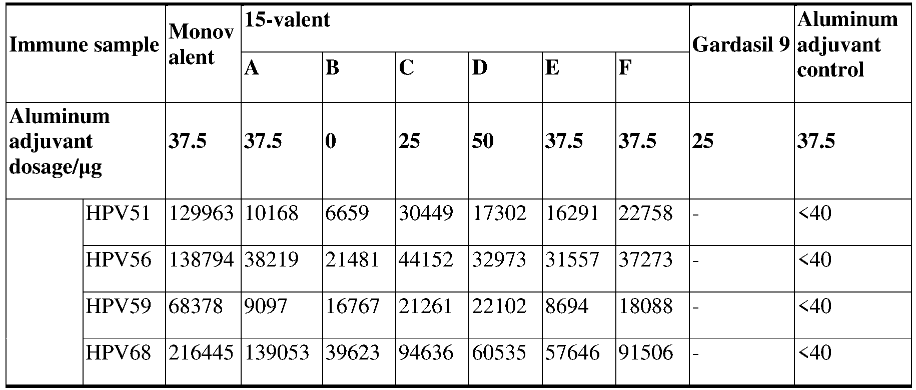

### Embodiment 4: Preparation process of 15-valent HPV vaccine

An HPV6/11/16/18/31/33/35/39/45/51/52/56/58/59/68 VLP bulk protein solution was taken from a refrigerator at -65°C to -80°C. The minimum storage concentration of each type of bulk protein solution was 1.56 mg/mL, and the pH range of a buffer used was 4.3-5.8 (see Embodiment 1). The buffer prescription included 20 mM HAc-NaAc, 500-700 mM NaCl, and 0.02% Tween 80. After being placed at room temperature for 10 min, the stock solution was thawed in a water bath at 37°C until completely melted. An aluminum hydroxide adjuvant was diluted to 4 mg/mL, and bacterial endotoxin, sterility, osmotic pressure molar concentration and aluminum content were detected. A density of the 4.0 mg/mL aluminum hydroxide adjuvant was 1.01 g/mL. 3120 mL of a filtered sample of type 15 protein diluent (with a density of 1.02 g/mL) and 3120 mL of aluminum hydroxide adjuvant diluent (with a density of 1.01 g/mL) were respectively taken, and mixed in a 10 L glass upright bottle (a magnetic stirrer with a rotor diameter of 1.39 cm and a length of 8.6 cm); and the resulting mixture was mixed well at 300-340 rpm for at least 30 min. A pre-filled syringe filling machine was started, which was set and operated according to the equipment operation and use regulations. The product was filled at a filling amount of 0.55 mL per vial for subpackaging, and plugged with a rubber stopper, with a filling speed of 30-40 rpm. The subpackaged products were labeled and stored.

### Embodiment 5: Further study on antigen dosage ratios

In order to obtain more research data on antigen dosage ratios and provide reference for vaccine dosages in clinical trials, a further study on antigen dosage ratios was conducted. 15-valent HPV vaccines with different antigen dosage ratios were prepared, BALB/c mice were immunized with 1/50× of the human dose, and the immunogenicity differences under different antigen dosage ratios were compared. The animal grouping information and antigen dosage ratio information are shown in Table 4. The vaccination program adopted 0 and 4 weeks of immunization, and the neutralizing antibody titers were measured at 4 weeks after the first immunization and at 4 weeks after the second immunization. Comparison between groups was carried out to determine the dosage ratios of various types of antigens in the vaccines.

**Table 4: Animal grouping table for restudy on antigen dosage ratios**

| **Group** | **Immune sample (1/50×)** | **Antigen dose for each type of HPV/µg** | | | | | | | | | | | | | | | **Alumi nium adjuva nt /µg** | **Number of animals** |
|---|---|---|---|---|---|---|---|---|---|---|---|---|---|---|---|---|---|---|
| | | **6** | **11** | **16** | **18** | **31** | **33** | **45** | **52** | **58** | **35** | **39** | **51** | **56** | **59** | **68** | | |
| Group 1 | 15-valent low dose | 0.6 | 0.8 | 1.2 | 0.8 | 0.4 | 0.4 | 0.4 | 0.4 | 0.4 | **0.2** | **0.2** | **0.2** | **0.2** | **0.2** | **0.2** | 10 | 10 |
| Group 2 | 15-valent medium dose | 0.6 | 0.8 | 1.2 | 0.8 | 0.4 | 0.4 | 0.4 | 0.4 | 0.4 | 0.4 | 0.4 | 0.4 | 0.4 | 0.4 | 0.4 | 10 | 10 |
| Group 3 | 15-valent high dose | 0.6 | 0.8 | **1.6** | 0.8 | 0.4 | 0.4 | 0.4 | 0.4 | 0.4 | 0.4 | 0.4 | 0.4 | 0.4 | 0.4 | 0.4 | 10 | 10 |
| Group 4 | 9-valent vaccine | 0.6 | 0.8 | 1.2 | 0.8 | 0.4 | 0.4 | 0.4 | 0.4 | 0.4 | - | - | - | - | - | - | 15 | 10 |
| Group 5 | Gardasil 9 | 0.6 | 0.8 | 1.2 | 0.8 | 0.4 | 0.4 | 0.4 | 0.4 | 0.4 | - | - | - | - | - | - | 10 | 10 |

**Table 5 Summary table of immune doses and neutralizing antibody geometric mean titers**

| **Immune sample** | **15-valent HPV vaccine** | | | | **KLWS 9** | **Gardasil 9** |
|---|---|---|---|---|---|---|
| | **Low dose** | **Medium dose** | | **High dose** | | |
| **Aluminum adjuvant dosage/**µg | **10** | **10** | | **10** | **15** | **10** |
| | HPV6 | 0.6 | 0.6 | 0.6 | 0.6 | 0.6 |
| | HPV11 | 0.8 | 0.8 | 0.8 | 0.8 | 0.8 |
| | HPV16 | 1.2 | 1.2 | 1.6 | 1.2 | 1.2 |
| | HPV18 | 0.8 | 0.8 | 0.8 | 0.8 | 0.8 |
| | HPV31 | 0.4 | 0.4 | 0.4 | 0.4 | 0.4 |
| | HPV33 | 0.4 | 0.4 | 0.4 | 0.4 | 0.4 |
| Antigen dosage/µg | HPV35 | 0.2 | 0.4 | 0.4 | - | - |
| | HPV39 | 0.2 | 0.4 | 0.4 | - | - |
| | HPV45 | 0.4 | 0.4 | 0.4 | 0.4 | 0.4 |
| | HPV51 | 0.2 | 0.4 | 0.4 | - | - |
| | HPV52 | 0.4 | 0.4 | 0.4 | 0.4 | 0.4 |
| | HPV56 | 0.2 | 0.4 | 0.4 | - | - |
| | HPV58 | 0.4 | 0.4 | 0.4 | 0.4 | 0.4 |
| | HPV59 | 0.2 | 0.4 | 0.4 | - | - |
| | HPV68 | 0.2 | 0.4 | 0.4 | - | - |
| Geometric mean titer of neutralizing antibody at 4 weeks after the first immunizatio n | HPV6 | 1658 | 2266 | 1617 | 1521 | 1655 |
| | HPV11 | 277 | 369 | 223 | 650 | 768 |
| | HPV16 | 4110 | 4263 | 4281 | 3636 | 5812 |
| | HPV18 | 6307 | 7175 | 7243 | 5350 | 6215 |
| | HPV31 | 2172 | 1861 | 1747 | 882 | 1549 |
| | HPV33 | 1190 | 2039 | 1504 | 1030 | 224 |
| | HPV35 | 504 | 883 | 337 | - | - |
| | HPV39 | 2593 | 1901 | 1576 | - | - |
| | HPV45 | 132 | 220 | 79 | 35 | 43 |
| | HPV51 | 135 | 295 | 335 | - | - |
| | HPV52 | 3268 | 1849 | 1846 | 2377 | 3169 |
| | HPV56 | 440 | 504 | 858 | - | - |
| | HPV58 | 2804 | 2154 | 2791 | 1570 | 4133 |
| | HPV59 | 394 | 825 | 887 | - | - |
| | HPV68 | 830 | 780 | 1485 | - | - |
| Geometric mean titer of neutralizing antibody at 4 weeks after the second immunizatio n | HPV6 | 15012 | 13287 | 14303 | 13075 | 17396 |
| | HPV11 | 4742 | 4124 | 3571 | 3291 | 5056 |
| | HPV16 | 35744 | 36411 | 45321 | 24459 | 45390 |
| | HPV18 | 32061 | 35805 | 34396 | 29418 | 35884 |
| | HPV31 | 15777 | 13912 | 12994 | 8703 | 17158 |
| | HPV33 | 15884 | 16486 | 18837 | 11015 | 5377 |
| | HPV35 | 4830 | 7019 | 4805 | - | - |
| | HPV39 | 9033 | 18440 | 15685 | - | - |
| | HPV45 | 3302 | 2026 | 1621 | 470 | 516 |
| | HPV51 | 2683 | 5292 | 5885 | - | - |
| | HPV52 | 19183 | 11822 | 20949 | 15230 | 13943 |
| | HPV56 | 6847 | 8215 | 17885 | - | - |
| | HPV58 | 39429 | 34836 | 43079 | 19438 | 38796 |
| | HPV59 | 4370 | 9040 | 6479 | - | - |
| | HPV68 | 9633 | 12482 | 17833 | - | |

Geometric means of neutralizing antibody detection at 4 weeks after the first immunization and at 4 weeks after the second immunization are shown in Table 5.
(1) Neutralizing antibody detection results at 4 weeks after the first immunization show that:
   Compared with the same types contained in Gardasil 9, the types with significant differences in the low-dose group are HPV11, 33, and 45, with HPV11 in Gardasil 9 being higher than that in a 15-valent HPV vaccine, and HPV33 and 45 in the 15-valent HPV vaccine being higher than those in Gardasil 9. The types with significant differences in the medium-dose group are HPV33 and 45, both of which in the 15-valent HPV vaccine are higher than those in Gardasil 9. The types with significant differences in the high-dose group are HPV11 and 33, with HPV11 in Gardasil 9 being higher than that in the 15-valent HPV vaccine, and HPV33 in the 15-valent HPV vaccine being higher than that in Gardasil 9. The levels of other types in the three dose groups are comparable, with no significant differences. The six new types (HPV35, 39, 51, 56, 59, and 68) added to this vaccine can induce strong immune responses in the low, medium, and high dose groups.
   There are significant differences between the low, medium, and high dose groups, including HPV45 (with a medium dose being higher than a high dose) and HPV59 (with a high dose being higher than a low dose). Compared with the medium dose group, the doses of HPV35, 39, 51, 56, 59 and 68 antigens in the low dose group are reduced by half, and there is no significant difference in neutralizing antibody levels. Compared with the medium dose group, the HPV16 antigen in the high dose group is increased from 60 µg/dose to 80 µg/dose, and there is no significant difference in neutralizing antibody titer level.
(2) Neutralizing antibody detection results at 2 weeks after the second immunization show that:
   Compared with the same types contained in Gardasil 9, the type with significant differences in the low-dose group is HPV 45, which is higher in a 15-valent HPV vaccine than in Gardasil 9. The type with significant differences in the medium-dose group is HPV33, which is higher in the 15-valent HPV vaccine than in Gardasil 9. The type with significant differences in the high-dose group is HPV 33, which is higher in the 15-valent HPV vaccine than in Gardasil 9. The levels of other types in the three dose groups are comparable, with no significant differences. The six new types (HPV35, 39, 51, 56, 59, and 68) added to this vaccine can induce strong immune responses in the low, medium, and high dose groups, resulting in high levels of neutralizing antibodies.

There are significant differences between the low, medium, and high dose groups, including HPV56 (with a high dose being higher than a low dose) and HPV59 (with a medium dose being higher than a low dose). Compared with the medium dose group, the doses of HPV35, 39, 51, 56, 59 and 68 antigens in the low dose group are reduced by half, and the induced neutralizing antibody levels are all lower than those in the medium dose group, but only the HPV59 shows a significant difference. Compared with the medium dose group, the HPV16 antigen in the high dose group is increased from 60 µg/dose to 80 µg/ dose, and the neutralizing antibody titer levels have increased but do not reach a significant difference.

Reducing the doses of HPV35, 39, 51, 56, 59, and 68 antigens based on the medium dose of the 15-valent HPV vaccine will reduce the immune response levels of these six types to a certain extent, but has no significant effect on the immune response of the other nine types.

Increasing the dose of the HPV16 antigen to 80 µg/dose on the basis of the medium dose of the 15-valent HPV vaccine can improve the immune response level of this type to a certain extent, making it closer to Gardasil 9, but there is no significant difference. Subsequent clinical trials need to comprehensively consider the results of major preclinical pharmacodynamic studies and the safety evaluation results to formulate dosage ratios of experimental vaccines.

Through the above research, it is determined that the relevant characteristics of this product preparation are briefly described as follows:
The adjuvant used is the commonly used aluminum hydroxide adjuvant, and its particle size distribution is approximately 1-20 µm measured by a laser particle size analyzer. It appears milky white in an aqueous solution and will precipitate after being placed for a certain period of time. After the protein antigen is adsorbed on the surface of the aluminum hydroxide adjuvant, the particle size of the aluminum hydroxide adjuvant may increase slightly, and the appearance thereof is basically similar to that of an aluminum hydroxide adjuvant without protein adsorption.

In the preparation process, 10 mM histidine was added to adjust the pH value from about 5.0 of the stock solution to about 6.0 of the preparation, which made it more acceptable to human body and reduced irritation. The results of a storage stability study showed that the pH value of the preparation was controlled within a range of 5.3-6.5 (including a histidine prescription), and there was no significant difference in the immunogenicity of the vaccine after the vaccine was placed at 37°C for 2 weeks.

The sodium chloride concentration in the preparation was set at 327 mM. The results of the storage stability study showed that setting the sodium chloride concentration of the preparation within a range 154-500 mM had no significant effect on the immunogenicity of the vaccine.

The dosage of the aluminum adjuvant in the preparation is 0.5 mg/dose, which is the same as the aluminum content of the commercially available 9-valent vaccine Gardasil 9. The difference is that the aluminum adjuvant used in Gardasil 9 is aluminum hydroxyphosphate sulfate adjuvant (belonging to one of aluminum phosphate adjuvants), rather than aluminum hydroxide. The selection of aluminum adjuvant types is mainly related to the properties of charges carried by antigens. The various types of proteins in the vaccines are prepared by expression after C-terminus truncation, with the parts where positive charges are enriched being removed. The proteins are negatively charged as a whole in pH close to neutral, while Gardasil9 uses a full-length sequence for protein expression and preparation, and the protein is positively charged as a whole in pH close to neutral. Negatively charged antigens are more easily adsorbed by positively charged aluminum hydroxide adjuvants, while positively charged antigens are more easily adsorbed by negatively charged aluminum phosphate adjuvants. In the vaccine preparations provided by the present invention, the adsorption rates of the aluminum hydroxide adjuvant to all the types of antigens are all greater than 95%, close to 100%. After the antigens are adsorbed on the surface of the aluminum adjuvant, it is not easy for the antigens to aggregate, which is beneficial to their stability.

The vaccines provided by the present invention are sterile preparations, need to be stored at 2-8°C, and cannot be frozen. The freezing process will destroy the crystal structure of the aluminum adjuvant, thus affecting its sedimentation properties, antigen adsorption capacity, and the like, and further affecting vaccine efficacy.

Finally, it should be noted that the foregoing descriptions are merely preferred embodiments of the present invention, and are not intended to limit the present invention. Although the present invention has been described in detail with reference to the aforementioned embodiments, those skilled in the art may still modify the technical solutions described in the aforementioned various embodiments, or equivalently replace some of the technical features therein. Any modification, equivalent replacement, improvement and the like made within the spirit and principle of the present invention shall be included within the protection scope of the present invention.

## Claims

1. A human papillomavirus (HPV) vaccine, **characterized in that** the vaccine comprises one or more combinations of L1 antigens selected from HPV types 6, 11, 16, 18, 31, 33, 35, 39, 45, 51, 52, 56, 58, 59, and 68, wherein the combination is 15 types, 9 types, 6 types, or 3 types of the L1 antigens;
wherein a weight ratio of amounts of the L1 antigens of HPV types 6, 11, 16, 18, 31, 33, 35, 39, 45, 51, 52, 56, 58, 59, and 68 is (1-3):(1-5):(2-7):(1-5):(0.5-3):(0.5-3):(0.5-3):(0.5-3):(0.5-3):(0.5-2):(0.5-2):(0.5-2):(0.5-2):(0.5-2):(0.5-2); and wherein the weight ratio of the amounts of the L1 antigens of HPV types 6, 11, 16, 18, 31, 33, 35, 39, 45, 51, 52, 56, 58, 59, and 68 is 1.5:2:3:2:1:1:1:1:1:1:1:1:1:1:1, or 1.5:2:3.5:2.5:1:1:1:1:1:1:1:1:1:1:1, or 3:4:6:4:2:2:2:2:2:1:1:1:1:1:1; and
wherein the amount of each protein is 10-100 µg, and wherein per 0.5 mL the vaccine contains: HPV type 6 at 30 µg, HPV type 11 at 40 µg, HPV type 16 at 60 µg, HPV type 18 at 40 µg, HPV type 31 at 20 µg, HPV type 33 at 20 µg, HPV type 35 at 20 µg, HPV type 39 at 20 µg, HPV type 45 at 20 µg, HPV type 51 at 20 µg, HPV type 52 at 20 µg, HPV type 56 at 20 µg, HPV type 58 at 20 µg, HPV type 59 at 20 µg, and HPV type 68 at 20 µg.

2. The vaccine according to claim 1, **characterized in that** each of the L1 antigens is truncated relative to the wild-type sequence as follows: wild-type HPV6 L1 is truncated by 2 amino acids at the N-terminus and by 29 amino acids at the C-terminus; wild-type HPV11 L1 is truncated by 3 amino acids at the N-terminus and by 29 amino acids at the C-terminus; wild-type HPV16 L1 is truncated by 4 amino acids at the N-terminus and by 29 amino acids at the C-terminus; wild-type HPV18 L1 is truncated by 4 amino acids at the N-terminus and by 30 amino acids at the C-terminus; wild-type HPV31 L1 is truncated by 4 amino acids at the N-terminus and by 27 amino acids at the C-terminus; wild-type HPV33 L1 is truncated by 4 amino acids at the N-terminus and by 24 amino acids at the C-terminus; wild-type HPV35 L1 is truncated by 4 amino acids at the N-terminus and by 28 amino acids at the C-terminus; wild-type HPV39 L1 is truncated by 9 amino acids at the N-terminus and by 29 amino acids at the C-terminus; wild-type HPV45 L1 is truncated by 4 amino acids at the N-terminus and by 30 amino acids at the C-terminus; wild-type HPV51 L1 is truncated by 4 amino acids at the N-terminus and by 28 amino acids at the C-terminus; wild-type HPV52 L1 is truncated by 4 amino acids at the N-terminus and by 23 amino acids at the C-terminus; wild-type HPV56 L1 is truncated by 4 amino acids at the N-terminus and by 25 amino acids at the C-terminus; wild-type HPV58 L1 is truncated by 4 amino acids at the N-terminus and by 23 amino acids at the C-terminus; wild-type HPV59 L1 is truncated by 4 amino acids at the N-terminus and by 31 amino acids at the C-terminus; and wild-type HPV68 L1 is truncated by 4 amino acids at the N-terminus and by 28 amino acids at the C-terminus.

3. The vaccine according to claim 1, **characterized in that** each of the L1 antigens comprises the amino acid sequence of SEQ ID NO: 1 to SEQ ID NO: 15, respectively.

4. The vaccine according to any one of claims 1 to 3, **characterized in that** an adjuvant used is an aluminum adjuvant, wherein the adjuvant is an aluminum hydroxide adjuvant; and a mass ratio of antigen protein to the aluminum adjuvant is (0.5-1):1, wherein the mass ratio is (0.7-0.85):1.

5. The vaccine according to any one of claims 1 to 3, **characterized in that** a buffer used in the vaccine is an acetic acid-sodium acetate buffer system, and the vaccine comprises an excipient composed of histidine, sodium chloride, and polysorbate 80.

6. The vaccine according to claim 5, **characterized in that** a pH value of the acetic acid-sodium acetate buffer system is 5-7, wherein the pH value is 5.3-6.5; a concentration of the acetic acid-sodium acetate buffer system is 5-15 mM, wherein the concentration is 10 mM; a concentration of sodium chloride is 100-400 mM, wherein the concentration is 320-335 mM; and a concentration of polysorbate 80 is 0.005%-0.02%, wherein the concentration is 0.01%.

7. A method for preparing the vaccine according to any one of claims 1 to 6, comprising the following steps:
purifying L1 proteins of each HPV type, subjecting the purified proteins to self-assembly *in vitro* to form virus-like particles (VLPs), subjecting the VLPs to column chromatography for buffer exchange and sterile filtration to prepare a bulk protein solution, and diluting the bulk protein solution with a buffer solution to a required concentration; and mixing the diluted protein solutions of all types in proportion to form a 15-valent protein dilution, which are sterilized and filtered for later use;
taking a prescribed amount of aluminum hydroxide adjuvant, diluting and mixing the aluminum hydroxide adjuvant uniformly (using a magnetic stirrer at 300-340 rpm for not less than 30 minutes), filtering the diluted aluminum hydroxide adjuvant through a capsule filter for later use;
taking a required amount of the sterile-filtered 15-valent protein dilution, taking a required amount of the filtered aluminum hydroxide adjuvant dilution, and mixing the protein dilution and adjuvant dilution until completely uniform to obtain the vaccine; and
wherein the method further comprising: performing final product filling by: starting a pre-filled syringe filling machine, operating the filling machine at 30-40 rpm, filling 0.55 mL per syringe and stoppering, and storing the stoppered syringes in cold storage for later use.

8. The method for preparing the vaccine according to claim 7, **characterized in that** the method for preparing the purified L1 proteins of each HPV type comprising:
taking a fermentation product of recombinant bacteria expressing the L1 protein of each type, resuspending bacterial cells from the fermentation product, disrupting the resuspended cells using a high-pressure homogenizer, centrifuging the disrupted cell slurry, and collecting a supernatant after centrifugation;
adding ammonium sulfate powder to the supernatant to a saturation of 25-45%, and slowly stirring until the resulting mixture is completely dissolved;
centrifuging continuously, collecting a precipitate, completely resuspending the precipitate, centrifuging the resuspended precipitate, and collecting a supernatant;
filtering the clarified supernatant, performing 2-stage depth filtration, and using filter membranes with pore sizes of 3.0 to 6.0 µm and 0.2 to 0.4 µm; and
performing EQ anion exchange chromatography→SQ anion exchange chromatography→gel filtration chromatography for optimization; wherein the specific operation comprises:
(1) EQ anion exchange chromatography: after column equilibration, loading a sample collected after deep filtration; performing a wash after loading completion, and collecting an EQ ion exchange flow-through liquid with OD280 greater than 50 mAU;
(2) SQ anion exchange chromatography: after column equilibration, loading the EQ ion exchange flow-through liquid from the step (1); performing a wash after loading completion; performing an elution after the wash ends, and collecting a target protein absorption peak with OD280 not less than 40 mAU during elution; and
(3) gel filtration chromatography: after equilibrating a chromatographic column until a baseline is stable, loading a target protein sample collected from SQ anion exchange chromatography elution, and collecting an HPV L1 protein absorption peak with OD280 greater than 50 mAU.

9. A method for preserving the vaccine according to any of claims 1 to 6, **characterized in that** the vaccine is preserved at 2-8°C.

10. A use of the vaccine according to any one of claims 1 to 6 in the preparation of a drug for preventing or treating diseases caused by human papillomavirus.
